# EUROPEAN PATENT SPECIFICATION

(11) **EP 1 560 853 B1**
(45) Date of publication and mention of the grant of the patent: **06.01.2010**
(21) Application number: 03776984.1
(22) Date of filing: 14.11.2003
(51) Int. Cl.: C07K 16/32, C07K 16/00, C12N 15/13, C12N 15/85, G01N 33/53

(54) **INTRACELLULAR ANTIBODIES**
INTRAZELLULÄRE ANTIKÖRPER
ANTICORPS INTRACELLULAIRES

(30) Priority: 15.11.2002 GB 0226729
(43) Date of publication of application: 10.08.2005
(73) Proprietor: MEDICAL RESEARCH COUNCIL, London W1B 1AL (GB)
(72) Inventor: RABBITTS, Terence Howard, Cambridge CB2 2QH (GB); TANAKA, Tomoyuki, Cambridge CB2 2QH (GB)
(74) Representative: Capasso, Olga
(86) International application number: PCT/GB2003/004942
(87) International publication number: WO 2004/046185

(56) References cited:
- WO-A-00/54057
- WO-A-03/077945
- US-A- 5 919 650
- DE JAEGER GEERT ET AL: "Analysis of the interaction between single-chain variable fragments and their antigen in a reducing intracellular environment using the two-hybrid system" FEBS LETTERS, vol. 467, no. 2-3, 11 February 2000 (2000-02-11), pages 316-320, XP004260975 ISSN: 0014-5793
- VISINTIN M ET AL: "Selection of antibodies for intracellular function using a two-hybrid in vivo system." PROCEEDINGS OF THE NATIONAL ACADEMY OF SCIENCES OF THE UNITED STATES OF AMERICA. 12 OCT 1999, vol. 96, no. 21, 12 October 1999 (1999-10-12), pages 11723-11728, XP002143442 ISSN: 0027-8424
- TANAKA TOMOYUKI ET AL: "Single domain intracellular antibodies: A minimal fragment for direct in vivo selection of antigen-specific intrabodies." JOURNAL OF MOLECULAR BIOLOGY, vol. 331, no. 5, 29 August 2003 (2003-08-29), pages 1109-1120, XP004447480 ISSN: 0022-2836
- DER MAUR ADRIAN AUF ET AL: "Direct in vivo screening of intrabody libraries constructed on a highly stable single-chain framework." THE JOURNAL OF BIOLOGICAL CHEMISTRY. 22 NOV 2002, vol. 277, no. 47, 22 November 2002 (2002-11-22), pages 45075-45085, XP002289622 ISSN: 0021-9258
- HARMSEN M.M.; SMITS C.B.; DE GEUS B.: 'Stimulation of chymosin secretion by simultaneous expression with chymosin-binding llama single-domain antibody fragments in yeast' APPL. MICROBIOL. BIOTECHNOL. vol. 60, 12 October 2002, pages 449 - 454
- MUYLDERMANS S.: 'Single domain camel antibodies: current status' REVIEWS IN MOLECULAR BIOTECHNOLOGY vol. 74, June 2001, pages 277 - 302, XP001057480
- WARD S.E. ET AL: 'Binding activities of a repertoire of single immunoglobulin variable domains secreted from Escherichia coli' NATURE vol. 341, 12 October 1989, LONDON, GB, pages 544 - 546, XP000086104
- COCHET O. ET AL: 'Intracellular expression of an antibody fragment-neutralizing p21 ras promotes tumor regression' CANCER RESEARCH vol. 58, 15 March 1998, pages 1170 - 1176, XP008067694
- TSE E. ET AL: 'Intracellular antibody capture technology: application to selection of intracellular antibodies recognising the BCR-ABL oncogenic protein.' J. MOL. BIOL. vol. 317, 15 March 2002, pages 85 - 94, XP004479165

## Description

The present invention relates to intracellular single domain antibodies and intracellular single domain antibody libraries, as well as methods for making and using such antibodies and antibody libraries.

Intracellular antibodies or intrabodies have been demonstrated to function in antigen recognition in the cells of higher organisms (reviewed in Cattaneo, A. & Biocca, S. (1997) Intracellular Antibodies: Development and Applications. Landes and Springer-Verlag). This interaction can influence the function of cellular proteins which have been successfully inhibited in the cytoplasm, the nucleus or in the secretory pathway. This efficacy has been demonstrated for viral resistance in plant biotechnology (Tavladoraki, P., et al. (1993) Nature 366: 469-472) and several applications have been reported of intracellular antibodies binding to HIV viral proteins (Mhashilkar, A.M., et al. (1995) EMBO J 14: 1542-51; Duan, L. & Pomerantz, RJ. (1994) Nucleic Acids Res 22: 5433-8; Maciejewski, J.P., et al. (1995) Nat Med 1: 667-73; Levy-Mintz, P., et al. (1996) J. Virol. 70: 8821-8832) and to oncogene products (Biocca, S., Pierandrei-Amaldi, P. & Cattaneo, A. (1993) Biochem Biophys Res Commun 197: 422-7; Biocca, S., Pierandrei-Amaldi, P., Campioni, N. & Cattaneo, A. (1994) Biotechnology (N Y) 12: 396-9; Cochet, O., et al. (1998) Cancer Res 58: 1170-6). The latter is an important area because enforced expression of oncogenes often occurs in tumour cells after chromosomal translocations (Rabbitts, T.H. (1994) Nature 372: 143-149). These proteins are therefore important intracellular therapeutic targets (Rabbitts, T.H. (1998) New Eng. J. Med 338: 192-194) which could be inactivated by binding with intracellular antibodies. Finally, the international efforts at whole genome sequencing will produce massive numbers of potential gene sequences which encode proteins about which nothing is known.

Functional genomics is an approach to ascertain the function of this plethora of proteins and the use of intracellular antibodies promises to be an important tool in this endeavour as a conceptually simple approach to knocking-out protein function directly by binding an antibody inside the cell.

We have recently described a technique for the selection of immunoglobulins which are stable in an intracellular environment, are correctly folded and are functional with respect to the selective binding of their ligand within that environment. This is described in international patent application WO00/54057. In this approach, the antibody-antigen interaction method uses antigen linked to a DNA-binding domain as a bait and the scFv linked to a transcriptional activation domain as a prey. Specific interaction of the two facilitates transcriptional activation of a selectable reporter gene. An initial in-vitro binding step is performed in which an antigen is assayed for binding to a repertoire of immunoglobulin molecules. Those immunoglobulins which are found to bind to their ligand in vitro assays are then assayed for their ability to bind to a selected antigen in an intracellular environment, generally in a cytoplasmic environment.

In our copending international patent application WO 2003/014960 we describe methods for producing intracellular immunoglobulins based on a consensus structure, optionally using the intracellular capture technique of WO00/54057,

The antigen binding domain of an antibody comprises two separate regions: a heavy chain variable domain (V_{H}) and a light chain variable domain (V_{L}: which can be either Vₖₐₚₚₐ or V_{lambda}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}. D and J_{H}. In humans, there are many functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the V_{H} domain (H3). The V_{L} gene is produced by the recombination of only two gene segments, V_{L} and J_{L}. In humans, there are approximately 40 functional V_{H} segments (Schäble and Zachau (1993) Biol. Chem. Hoppe-Seyer, 374: 1001), 31 functional V_{L} segments (Williams et al. 1996) J. Mol. Biol., 264: 220; Kawasaki et al. (1997) Genome Res., 7: 250), 5 functional Jₖₐₚₚₐ segments (Hieter et al. (1982) J. Biol. Chem., 257: 1516) and 4 functional J_{lambda} segments (Vasicek and Leder (1990) J. Exp. Med., 172: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

Single chain variable fragments, also known as scFv, which are composed of the heavy (H) and light (L) chain variable domains and a flexible linker peptide to create a single polypeptide chain, have been recognised as the most suitable form for ICAb because the normal association of free H and L chains occurs in the endoplasmic reticulum and will not occur in the cytoplasm of cells. ScFv, on the other hand, are single polypeptides in which V_{H} and V_{L} associate by intrinsic affinity and no inier-chain disulphide bonds are needed. Indeed, this format has been demonstrated to be effective against target proteins *in vivo* when selected according to the techniques previously described by the present inventors (see WO00/54057) but comparatively few scFv work efficiently as ICAb because of their insolubility, instability and incorrect protein folding in a reducing environment. The approaches described above overcome this limitation and direct screening based on intrinsic scFv in vivo folding and biological functions (intracellular antibody capture, IAC, WO00/54057) has proved successful in selecting ICAbs recognising a diverse set of antigens. In addition, IAC technology has helped to define a scaffold of immunoglobulin V-region residues which are particularly advantageous for in cell function WO/2003/014960).

A limitation of using scFv as the source of ICAb is the combinatorial effect of heavy and light chain and the subsequent diversity required for initial screening for antigen specific ICAbs. In typical screening protocols, diverse phage antibody libraries of greater than 10⁹ are needed to facilitate the isolation of a small number (around 10-50) of ICAbs. Moreover, the association of V_{H} and V_{L} domains is weak and the dissociated form of scFv can be dominant compared to associated form. Dissociated scFv are the target of proteolysis and aggregation inside cells. An alternative form of V_{H} -V_{L} heterodimer is disulphide-stabilised Fv fragments (dsFv), but this in not option for ICAb because the disulphide bond is not maintained inside cells.

Several efforts have been made to reduce the size of antibody fragments, for conventional in vitro use, even further. The smallest immunoglobulin-based recognition unit so far used are single variable domains (Ward *et al*.; Winter II). These so-called domain antibodies (Dabs) have been expressed in bacteria and functional V_{H} domains have been isolated from the libraries made from immunised mice.

Recently, several natural V_{H} fragments and heavy chain antibodies in absence of light chain found in camel and related species have been demonstrated to possess effective binding activity and specificity *in vitro*. Moreover, single domain libraries have been constructed by randomising CDR3 region of human V_{H} domain or mouse V_{H} domain, but these have been limited to *in vitro* applications, particularly as the framework may not be suitable for intracellular use.

### Summary of the Invention

Here we show intracellular V_{H} domain antibodies (IDabs), based on a consensus V_{H} framework derived from IAC of scFv, are highly efficacious against antigen in mammalian cells. A practical highlight is the generation of IDab libraries (with randomized CDRs based on the consensus scaffold framework) which are of sufficient diversity to allow direct selection in yeast of high affinity, antigen-specific antibodies.
These libraries have been applied successfully to isolate IDab with different antigen, viz. oncogene RAS and the cAMP/calcium dependent transcription factor ATF-2. The anti-RAS IDab can inhibit mutant RAS-induced NIH 3T3 cell transformation.

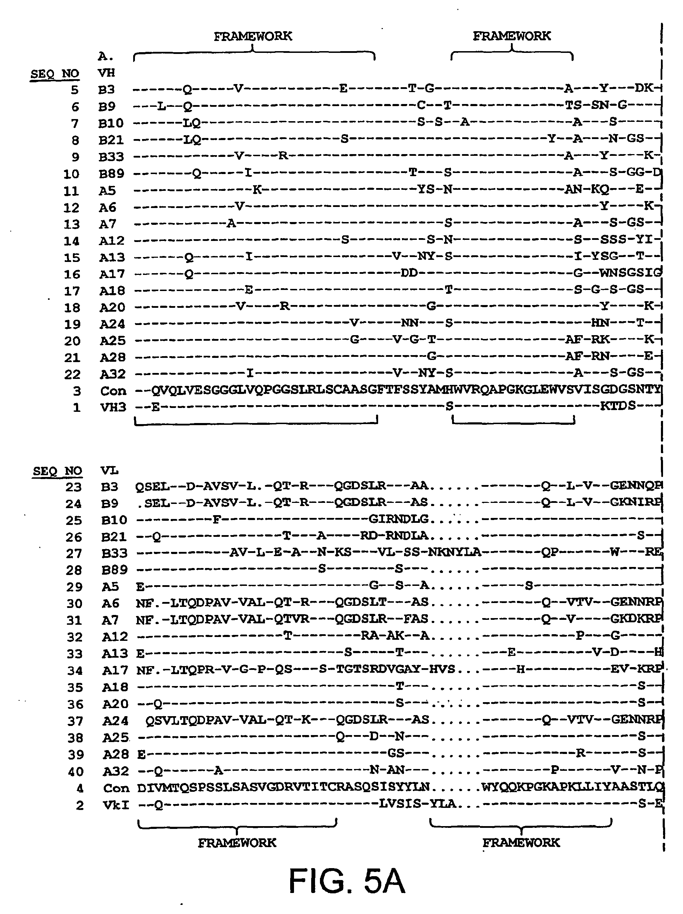

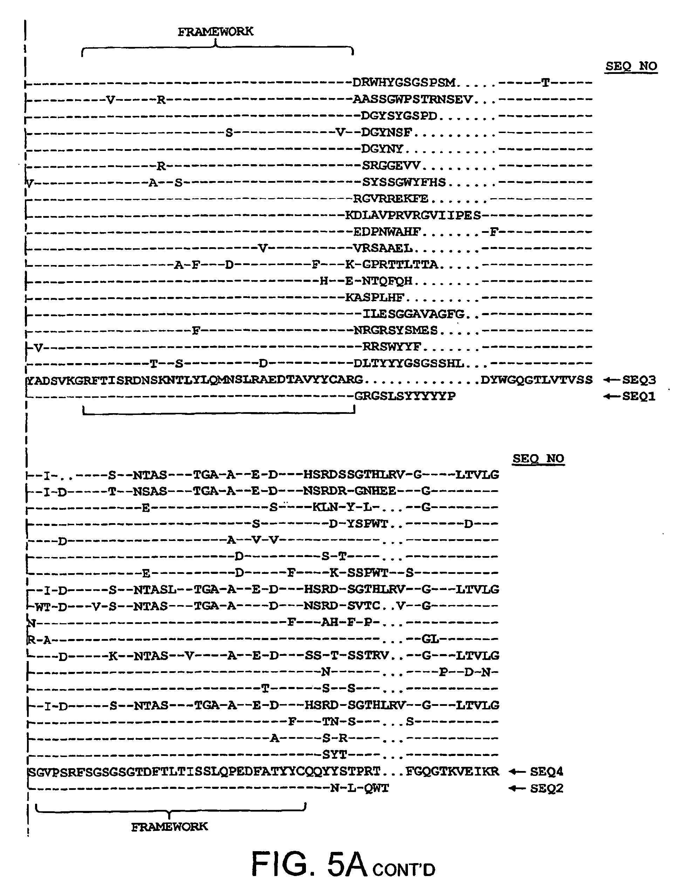

In accordance with the present invention, therefore, there is provided a method for determining the ability of a immunoglobulin V_{H} single domain to bind to a target in an intracellular environment, comprising the steps of:
a) providing a first molecule and a second molecule, wherein stable interaction of the first and second molecules leads to the generation of a signal;
b) providing a single intracellular V_{H} immunoglobulin domain which is associated with the first molecule, said single immunoglobulin V_{H} domain being free of complementary immunoglobulin domains;
c) providing an intracellular target which is associated with the second molecule, such that association of the immunoglobulin V_{H} domain and the target leads to stable interaction of the first and second molecules and generation of the signal;
d) assessing the intracellular interaction between the immunoglobulin domain and the target by monitoring the signal;
wherein the V_{H} domain exhibits at least 85% homology to the consensus sequence shown in Figure 5a and depicted as SEQ ID No 3.

The basis of the method of the present invention is that when the first and second molecules are brought into stable interaction by binding of immunoglobulin single domain to target in the intracellular environment, a signal is generated. The first and second molecules are thus two parts of a signal-generating agent which are capable of generating a signal by interacting. A "stable interaction" is an interaction which allows the generation of a signal through interaction between the first and second molecules. The degree of stability required will depend on the degree of such interaction which is required to generate a signal. For instance, if the signal is a biological event such as the reconstitution of a transcription factor and the induction of transcription, the stability will be required to be relatively high. However, if the signal is a signal such as a FRET interaction, the stability need only be relatively low. A "signal", as referred to herein, is any detectable event. This may include a luminescent, fluorescent or other signal which involves the modulation of the intensity or frequency of emission or absorption of radiation; for example, a FRET signal or the induction of a luciferase gene; these and other signals are further described below.

The immunoglobulin single domains are advantageously single domain antibodies. Antibodies according to the invention, referred to herein as intracellular domain antibodies or IDAbs, preferably comprise a single V_{H} or V_{L} domain the structure of which is suitable for intracellular binding of antigen, being able to maintain its specificity and correctly folded structure *in vivo* in an intracellular environment.

The advantages of single domain antibody fragments for intracellular use, compared with the scFv, are not only smaller size but also a higher stability. Furthermore, the smaller size of the single domain and the lack of any need to take V_{H} V_{L} interactions into consideration means the overall complexity for screening is lower than for scFv.

"Intracellular" means inside a cell, and the present invention is directed to the selection of immunoglobulin single domains which will bind to targets selectively within a cell. The cell may be any cell, prokaryotic or eukaryotic, and is preferably selected from the group consisting of a bacterial cell, a yeast cell and a higher eukaryote cell. Most preferred are yeast cells and mammalian cells. In general, the assay of the invention is carried out in the cytoplasm or nucleus of the cell, and determines the ability of the immunoglobulin to fold effectively within the cytoplasm or nucleoplasm and bind to its target. As used herein, therefore, "intracellular" immunoglobulins and targets are immunoglobulins and targets which are present or capable of functioning within a cell, preferably in the cytoplasm. Antibodies which are secreted into the Golgi or ER are not intracellular antibodies as defined herein.

In a further embodiment, the method of the invention may be conducted under conditions which resemble or mimic an intracellular environment. Thus, "intracellular" may refer to an environment which is not within the cell, but is *in vitro.* For example, the method of the invention may be performed in an *in vitro* transcription and/or translation system, which may be obtained commercially, or derived from natural systems. Preferably, the environment is adjusted such that the reducing conditions present in cellular cytoplasm are replicated, allowing for faithful selection of immunoglobulins capable of selective binding to targets in true intracellular conditions.

Advantageously, the method of the invention further comprises a functional assay for the immunoglobulin single domain. Thus, the method preferably further includes the step of selecting the immunoglobulins which cause a signal to be generated in the intracellular environment, and subjecting those immunoglobulins to a functional intracellular assay. For example, where the assay is intended to select immunoglobulins which bind to targets which are associated with tumourigenesis, such as the gene product of a mutant Ras oncogene, the immunoglobulins may be tested in a cell transformation assay to determine any modulating activity on the production of transformed cells.

The first and second molecules may be any molecules, consistent with the requirement to generate a signal. They need not necessarily be polypeptides. For example, they may be fluorophores or other chemical groups capable of emitting or absorbing radiation. In a preferred aspect, however, the first and second molecules of the invention are polypeptides.

Polypeptides according to the invention associate to form an active reporter molecule which is itself capable of giving a signal. Preferably, therefore, the polypeptides are domains of such a reporter molecule.

For example, the polypeptides may be domains of a fluorescent polypeptide, such as GFP, or domains of a transcription factor which, when active, upregulates transcription from a reporter gene. The reporter gene may itself encode GFP, or another detectable molecule such as luciferase, β-galactosidase, chloramphenicol acetyl transferase (CAT), an enzyme capable of catalysing an enzymatic reaction with a detectable end-point, or a molecule capable of regulating cell growth, such as by providing a required nutrient.

Association of the immunoglobulin and the target in accordance with the invention provides a stable link between the first and second molecules, which brings the molecules into stable interaction. "Stable interaction" may be defined as an interaction which permits functional cooperation of the first and second molecules in order to give rise to a detectable result, according to the signalling methods selected for use. Advantageously, a stable interaction between the first and second molecules does not occur unless the molecules are brought together through binding of the immunoglobulin and the target.

The terms "immunoglobulin" and "target" are used according to their ordinary signification given in the art, as further defined below. The term "immunoglobulin", in particular, refers to any moiety capable of binding a target, in particular a member of the immunoglobulin superfamily, including T-cell receptors and antibodies. It includes any fragment of a natural immunoglobulin which is capable of binding to a target molecule, for example antibody fragments such as Fv and scFv. The term "target" includes antigens, which may be targets for antibodies, T-cell receptors, or other immunoglobulin.

Preferably, the immunoglobulin is an antibody and the target is an antigen. An "antibody" single domain is a single V_{H} domain or a single V_{L} domain. Preferably, it is a single V_{H} domain.

As is known in the art, single domain antibodies may be "camelised" by mutating certain residues as the V_{H}-V_{L} interface to render the V_{H} (or V_{L}) domain less "sticky" and thus less prone to non-specific binding. See, for example, Riechmann et al. (1996) J.Mol.Biol 259:957-969. The present inventors have determined that "camelising" intracellular single domain antibodies reduces or eliminates the ability of the antibody to bind intracellularly. Thus, the immunoglobulins according to the invention are advantageously not camelised.

In a preferred embodiment, the immunoglobulin single domain and target are provided by expressing nucleic acids within the cell in which the intracellular assay is to take place. The immunoglobulin and target constructs, which comprise the signal-generating molecules, are transcribed and/or translated from nucleic acid and localised to, for instance, the cytoplasm of the cell, where the intracellular assay may take place. In other advantageous embodiments the intracellular immunoglobulins may be localised to any desired subcellular compartment, such as the nucleus (for example by fusion to a nuclear localisation signal), to the ER, using an ER retention signal, or other locations.

Nucleic acids encoding immunoglobulins may be obtained from libraries encoding a multiplicity of such molecules. For example, phage display libraries of immunoglobulin molecules are known and may be used in this process. Advantageously, the library encodes a repertoire of immunoglobulin domains. A "repertoire" refers to a set of molecules generated by random, semi-random or directed variation of one or more template molecules, at the nucleic acid level, in order to provide a multiplicity of binding specificities. Methods for generating repertoires are well characterised in the art.

Libraries may moreover be constructed from nucleic acids isolated from organisms which have been challenged with a target, for example an antigen. Antigen challenge will normally result in the generation of a polyclonal population of immunoglobulins, each of which is capable of binding to the antigen but which may differ from the others in terms of epitope specificity or other features. By cloning immunoglobulin genes from an organism a polyclonal population of immunoglobulins may be subjected to selection using the method of the invention in order to isolate immunoglobulins which are suitable for use in intracellular environment.

The method of the invention permits the isolation of immunoglobulin domains which are capable of intracellular binding activity, and/or nucleic acids encoding such immunoglobulins, on the basis of the signal generated by the method set forth above. Accordingly, one or both of the immunoglobulin domain and the target used in the method of the invention, together with the first or second molecules, are provided in the form of nucleic acid constructs which are transcribed to produce said immunoglobulin domain and/or target together with said first or second molecules. Nucleic acid constructs may be expression vectors capable of directing expression of the nucleic acid encoding the immunoglobulin domain in the cell in which the method of the invention is to be performed.

The present invention allows the isolation of immunoglobulin domains and/or nucleic acids encoding them which bind to targets intracellularly. Advantageously, the immunoglobulin domains which are screened by the method of the present invention are previously selected for target specificity. Accordingly, the invention provides a method for preparing an immunoglobulin single domain suitable for use in a procedure according to the invention, comprising the steps of:
a) expressing a repertoire of immunoglobulin single domain genes in a selection system and isolating those genes which encode immunoglobulin domains specific for a desired target;
b) bringing the isolated genes into operative association with nucleic acids encoding a first molecule, wherein stable interaction of the first molecule with a second molecule generates a signal, in order to produce a fusion polypeptide comprising the immunoglobulin domain and the first molecule.

As used above, "operative association" refers to the fusion or juxtaposition of coding sequences such that a fusion protein is produced, comprising the immunoglobulin domain and the signal-generating molecule. Normally, performing a selection against an target will generate a smaller repertoire of antibodies which share target specificity. The transcription units encoding such immunoglobulins, fused to the signal generating molecules, are employed in an assay according to the invention in order to select those immunoglobulin domains which are capable of functioning intracellularly.

In a further aspect, the invention provides a library of immunoglobulin single domains wherein each single domain is operatively associated with a first molecule of a reporter system as described above. Preferably, the library is a library of antibody single domains, which are advantageously V_{H} domains.

The present inventors have moreover determined that single domains derived from multidomain intracellular antibodies function highly efficiently as intracellular single domain immunoglobulins. Thus, the invention provides a method for preparing an intracellular single domain immunoglobulin which binds to a target in an intracellular environment, comprising the steps of:
a) providing a first molecule and a second molecule, wherein stable interaction of the first and second molecules leads to the generation of a signal;
b) providing a single intracellular V_{H} immunoglobulin domain which is associated with the first molecule, said single immunoglobulin V_{H} domain being free of complementary immunoglobulin domains;
c) providing an intracellular target which is associated with the second molecule, such that association of the immunoglobulin V_{H} domain and the target leads to stable interaction of the first and second molecules and generation of the signal;
d) assessing the intracellular interaction between the immunoglobulin domain and the target by monitoring the signal;
wherein the V_{H} domain exhibits at least 85% homology to the consensus sequence shown in Figure 5a and depicted as SEQ ID No 3.

Advantageously, the method further comprises the step of mutating the framework regions of the single domain immunoglobulin to enhance intracellular binding and/or stability.

Single domain antibodies functioning in intracellular environments have been shown herein not to require the intradomain disulphide bond commonly present in V_{H} domains. Advantageously, therefore, the intracellular single domains do not comprise an intradomain disulphide bond.

Single domain antibodies of the invention advantageously conform to the intracellular V_{H} or V_{L} consensus described in WO/2003/014960.

Advantageously, the consensus is described by at least one of the consensus sequence described in Figure5a and depicted SEQ ID no 3. Advantageously, the "consensus" used in the present invention is at least 85% identical to that shown in Figure5a and SEQ. ID. No. 3; preferably 90%, 95%, 96%, 97%, 98%, 99% or 100% identical thereto. Preferably, in the calculation of identity, the amino acid residues of CDR3 are excluded from consideration.

The invention moreover provides libraries of single domain antibodies as described above, wherein said libraries comprise single domain antibodies consisting of V_{H} domains which conform to the intracellular consensus, as described.

Intracellular single domain immunoglobulins according to the invention are useful in intracellular therapeutic applications. Accordingly, the invention provides a method for modulating a biological function in a cell comprising administering to the cell an effective amount of an intracellular single domain immunoglobulin as described. Moreover, the invention provides the use of an intracellular single domain according to the invention in the manufacture of a composition for the modulation of a biological function in a cell.

The biological function may be any desired function, including the upregulation and downregulation of gene expression at the polypeptide or nucleic acid level. For example, single domain intracellular immunoglobulins may specifically target oncogenic gene products, whether at the polypeptide or mRNA level, and downregulate their expression. For example, the oncogene may be an activated p21 Ras oncogene. Single domain immunoglobulins according to the invention have been shown to prevent oncogenic transformation by Ras oncogenes.

### Brief Description of the Figures

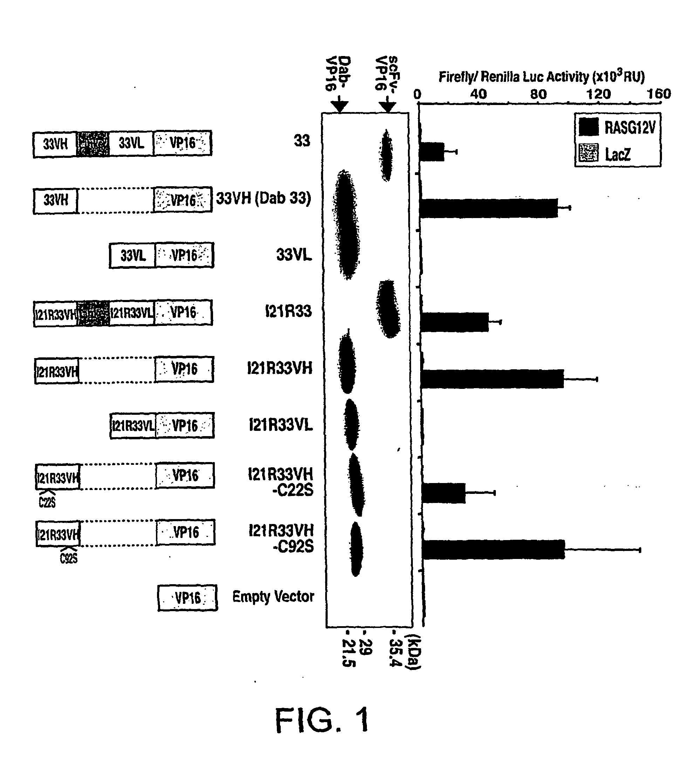

**Figure 1****. Interaction of anti-RAS scFv intrabodies and single domain derivatives with RAS protein in mammalian cells.**
   COS7 cells were transiently co-transfected with various pEF-VP16 expression clones synthesising scFv or single domain derivatives fused with the VP16 activation domain, together with the GAL4-DBD bait plasmid pM1-HRASG12V (closed black boxes) or pM1-ß-galactosidase (lacZ) (grey boxes), In addition, the firefly luciferase reporter plasmid pG5-Luc and an internal Renilla luciferase control plasmid pRL-CMV were co-transfected. The luciferase activities were measured 48 hours after transfection. In the right hand panel, the normalised activity of firefly luciferase signals to the Renilla luciferase activity (used as internal control for the transaction efficiency) are shown. The middle panel shows a Western blot of COS7 cell extracts after the expression of scFv-VP16 fusion proteins detected using anti-VP16 (14-5, Santa Cruz Biotechnology) monoclonal antibody and horseradish peroxidase (HRP)-conjugated anti-mouse IgG antibody. The left hand panel indicates the constructs used to express the various antibody fragments.

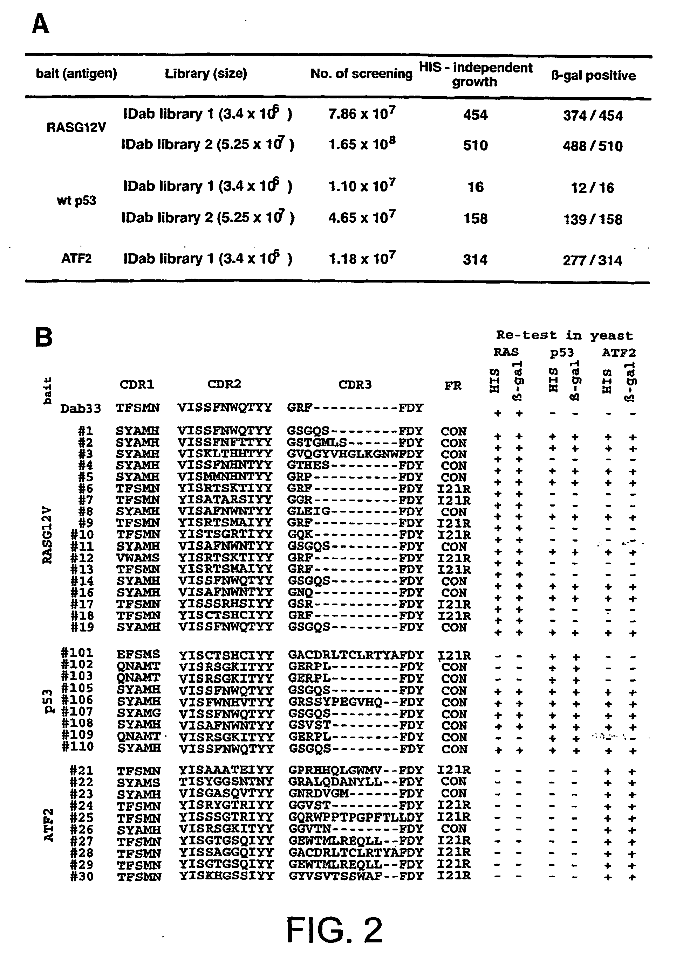

**Figure 2****. Intracellular antibody capture uisng synthetic single domain libraries.**
   **A**. The diveristy of the two pVP16*-Dab libraries Were: I21R33-derived library 1 2 x 10⁶ and consensus library 1 1.4 x 10⁶ (i.e. 3.4 X 10⁶ total diversity). The first library 1 pool was diversified at CDR1 as described in the methods. The respective diversities of library 2 was 3.04 x 10⁷ for I21R33-derived library and 2.215 x 10⁷ consensus-derived library (i.e. 5.25 x 10⁷ total diversity). 12 clones were randomly picked from each library and sequenced to verify the insert and the correct integration of CDRs. The primary screening results are shown for initial clones screened in yeast L40 and the numbers of colonies growing on histidine deficient plates with the three baits (RAS, p53 and ATF-2). The corresponding proportion causing β-gal activation are shown on the right column.
   **B.** Alignment of derived protein sequences of selected intracellular Dab obtained with the 3 baits. The nucleotide sequences were obtained and the derived protein translations (shown in the single-letter code) were aligned. The complementarity determining regions (CDR) (as defined by Kabat et al. ²⁶ and by IMGT ²⁷) are shown in left panel (only 11 residues at N-terminal of CDR2 are shown).
   The right panel shows the results of re-testing IDabs in an antigen-antibody interaction in yeast using different baits to verify the specificity of Dab with antigen. The HIS column shows histidine independent growth assay and β-gal, β-galactosidase filter assay.
   Clones 1-9, 21-30 came from the library 1 (either canonical consensus or I21R framework)
   Clone 11-19, 101-110 came from the library 2 (either canonical consensus or I21R framework)
   CON= composed of VH domain of consensus framework sequence ⁴
   I21R + VH domain of anti-RAS scFvI21R33 framework ⁹.

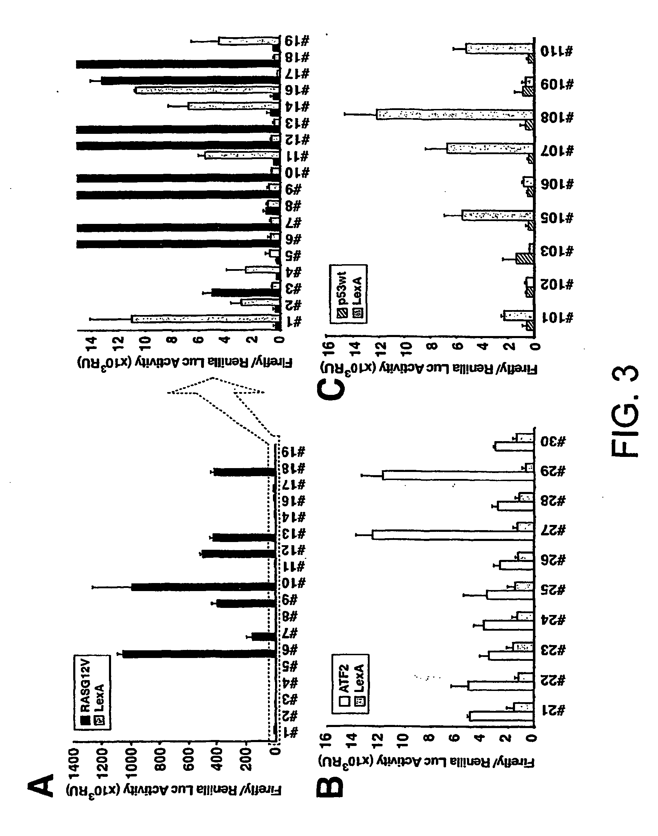

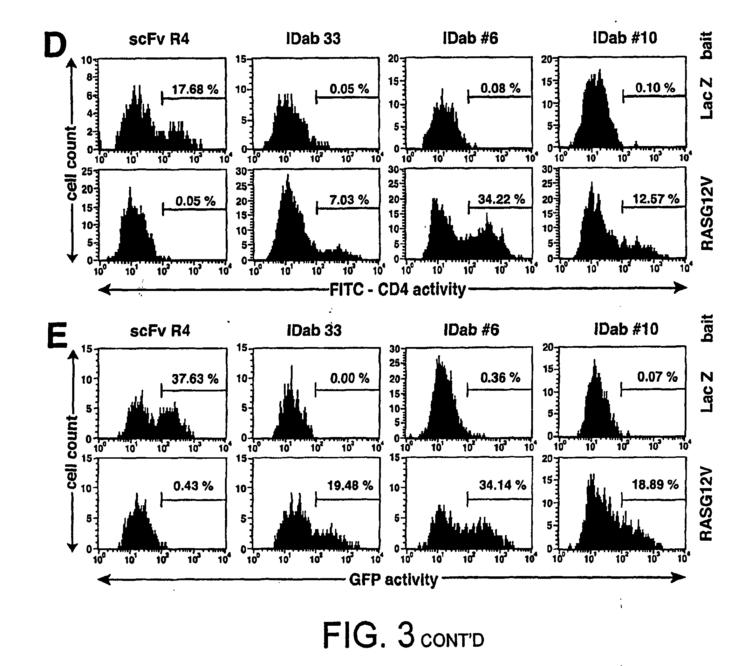

**Figure 3****. Interaction between single domain intrabodies and antigen in mammalian cells**
   Mammalian two-hybrid antibody-antigen interaction assays were performed in three independent methods.
   **A-C. Luciferase reporter assays;** COS7 were transfected with the pEF-IDab-VP16 vectors and the baits pMl-HRASG12V (closed black boxes), pMl-ATF-2 (open boxes), pMl-p53wt (diagonal boxes), or pMl-LexA (grey boxes) together with pG5-luc and pRL-CMV. Luciferase levels were determined as described in Figure 1. Each histogram represents activity of firefly luciferase signals normalised to the Renilla luciferase activity (used as internal control for the transfection efficiency). (A) RAS selected IDab subgroup isolated from IAC screening with RAS antigen. The top right histogram is focused to low range (up to 15 x 10⁻³ of activity ratio as full scale) of the left histogram (up to 1.4 as full scale). (B) ATF-2 selected IDab. (C) p53 selected IDab.
   **D. FACS analysis for CD4 expression.** The CHO-CD4 cells, carrying a CD4 reporter gene regulated via the Gal4 upstream activating sequence (UAS) site ¹⁴, were co-transfected with pMl-HRASG12V or pMl-lacZ together with various pEF-scFv-VP16 or pEF-IDab-VP16 vectors. Induction of cell surface CD4 expression was assayed at 48 h after transfection by using anti-human CD4 antibody and FITC-conjugated anti-mouse Ig. The indicated percentages of CD4+ cells were measured using a FACSCalibur.
   **E. FACS analysis for GFP expression.** A CHO-GFP cell line with a GFP reporter gene regulated via the GAL4 UAS ¹⁷ were co-transfected with pMl-HRASG12V or pMl-lacZ together with various pEF-scFv-VP16 or pEF-IDab-scFv vectors. 48 hour after transfection, cytoplasmic GFP expressions were measured using a FACSCalibur.

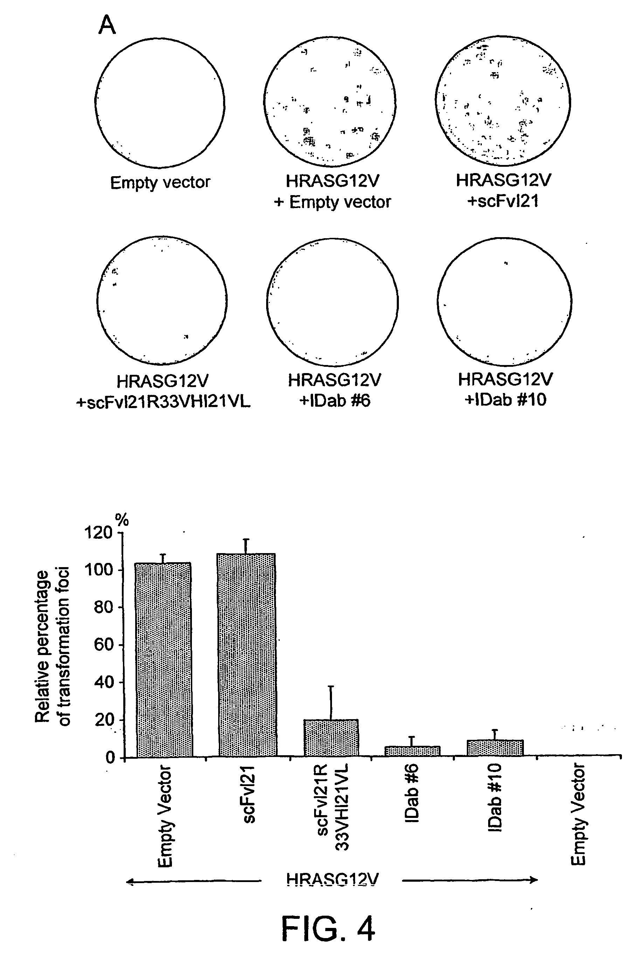

**Figure 4****. Inhibition of RAS-mediated oncogenic transformation of NIH3T3 cells by anti-RAS single domain intrabody.**
   Mutant HRASG12V cDNA were subcloned into the mammalian expression vector pZIPneoSV(X) and anti-RAS scFv or IDab into pEF-FLAG-Memb vector, which has plasma membrane targeting signal at C-terminal of scFv or IDab and a FLAG-tag at N-terminal ⁹. 100ng of pZIPneoSV(X)-HRASG12V and 2µg of pEF-Memb-scFv or pEF-Memb-IDab were co-transfected into low passage NIH3T3 cells cloneD4 using LipofectAMINE^{™} (Invitrogen) . Two days later, the cells were transferred to 10cm plates. After reaching confluence, cells were maintained for 14 days in Dulbecco's modified Eagle's medium containing 5% donor calf serum and penicillin and streptomycin. The plates were stained with crystal violet and foci of transformed cells were quantitated.
   **A.** Representative photograph of growth plates. Empty vector in top left panel indicates co-transfection of pZIPneoSV(X) without RAS and pEF-VP16 vector without scFV or IDab (negative control); no foci formation were observed. Other plates were from transfections of HRASG12V plus the indicated scFv or IDab
   **B.** Relative percentage of transformation foci in histogram was determined as a number of foci normalised to the focus formation induced by pZIPneoSV(X)-HRASG12V and pEF-VP16 empty vector, which was set at 100. Results shown represent one experiment in which each transfection was performed in duplicate (two additional experiments yield similar results).

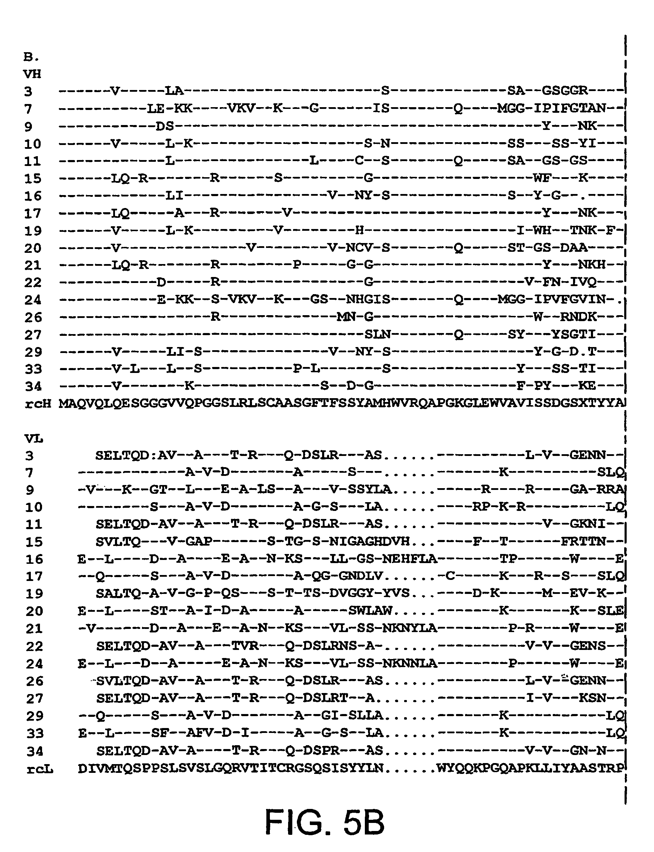

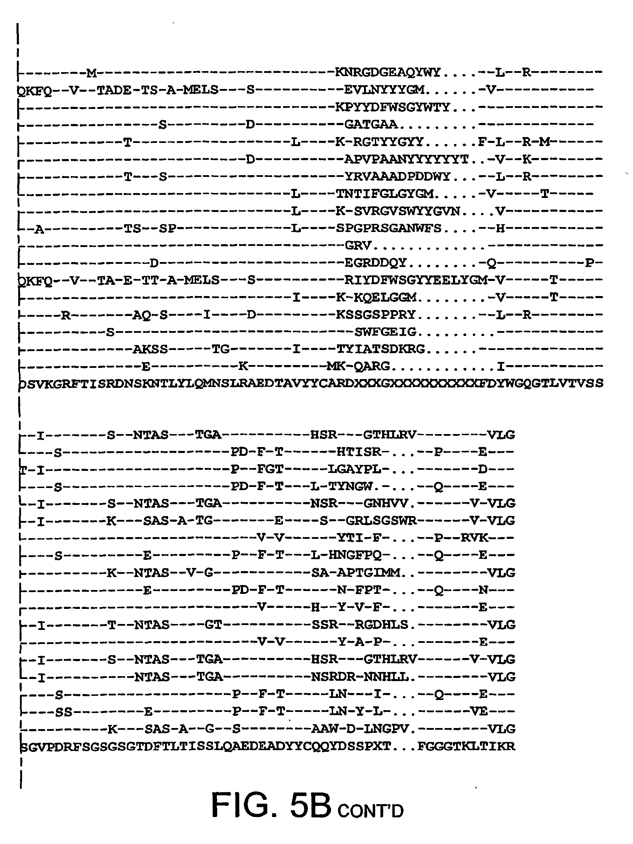

**Figure 5** **shows the Alignment of derived protein sequences of intracellular scFv.**
   The nucleotide sequences of the scFv were obtained and the derived protein translations (shown in the single letter code) were aligned. The complementarity determining regions (CDR) are shaded. Framework residues for SEQ no 1 to 40 are those which are underlined. The consensus sequence at a specific position was calculated for the most frequently occurring residue but only conferred if a residue occurred greater than 5 times at that position.
   A. Sequences of VH and VL from anti-BCR (designated as B3-B89) and anti-ABL (designated as A5-A32). The combined consensus (Con) of the anti-BCR and ABL ICAbs is indicated compared with the subgroup consensuses forVH3 and V_{K}I from the Kabat database.
      - Represents sequence identity with the intracellular antibody binding V_{H} or V_{L} consensus (SEQ. ID. No. 3 and SEQ. ID. No. 4 respectively) represents gaps introduced to optimise alignment
   B. A sequence comparison of randomly obtained scFv obtained from the unselected phage display library. The consensuses obtained from the randomly isolated scFv (rcH and rcL) are indicated.
      - represents gaps introduced to optimise alignment
      X represents positions at which no consensus could be assigned.

### Detailed Description of the Invention

### Definitions

Single domain immunoglobulins, according to the present invention, refer to any single domain moieties which are capable of binding to a target. In particular, they include single domains derived from members of the immunoglobulin superfamily, a family of polypeptides which comprise the immunoglobulin fold characteristic of antibody molecules, which contains two β sheets and, usually, a conserved disulphide bond. Members of the immunoglobulin superfamily are involved in many aspects of cellular and non-cellular interactions *in vivo*, including widespread roles in the immune system (for example, antibodies, T-cell receptor molecules and the like), involvement in cell adhesion (for example the ICAM molecules) and intracellular signalling (for example, receptor molecules, such as the PDGF receptor). The present invention is applicable to single domain molecules derived from all immunoglobulin superfamily molecules which are capable of binding to target molecules. Preferably, the present invention relates to antibody single domains, in particular heavy chain variable (V_{H}) domains. Single domain immunoglobulins are free of complementary domains, that is are not associated with other binding domains which, in nature or otherwise, may associate with the single domain to form a single composite binding site for a target. Specifically, V_{H} domains are not in the presence of complementary V_{L} domains in the single domains of the invention. However, further domains, such as antibody constant region domains, may be but need not be present.

A **domain** is used in its ordinary meaning in the art; thus, a domain (typically of a polypeptide or protein) possesses an independent tertiary structure and an independent functional attribute. Domains may be assembled to form multi-domain proteins.

**Antibodies,** as used herein, refers to complete antibodies or antibody fragments capable of binding to a selected target, and including Fv, ScFv, Fab' and F(ab')₂, monoclonal and polyclonal antibodies, engineered antibodies including chimeric, CDR-grafted and humanised antibodies, and artificially selected antibodies produced using phage display or alternative techniques. Antibodies may be or be based on of any naturally-occurring antibody type, including IgG, IgE, IgA, IgD and IgM. Single domains, such as V_{H} domains, may be derived from any such antibody.

A molecule is any chemical structure, including an inorganic molecule, an organic molecule or a combination of the two. Typically, the molecule will be a polypeptide or a nucleic acid. Polypeptides are chains of amino acids joined through peptide bonds, and may comprise natural or synthetic amino acids, or combinations of the two.

An **active reporter molecule** is a molecule which is capable of generating a signal, either directly or through a chemical or biological pathway. For example, an active reporter molecule may be a pair of fluorophores, which interact to generate a signal through FRET; or two domains of a transcription factor, which interact to form an active transcription factor; or domains of an enzyme, which interact to reconstitute a detectable enzyme activity.

**Heavy chain variable domain** refers to that part of the heavy chain of an immunoglobulin molecule which forms part of the antigen binding site of that molecule. The abbreviation V_{H} is used. Several subtypes, based on structural similarities, have been defined, for example as set forth in the Kabat database.

**Light-chain variable domain** refers to that part of the light chain of an immunoglobulin molecule which forms part of the antigen binding site of that molecule. The abbreviation V_{L} is used. Several subtypes, based on structural similarities, have been defined, for example as set forth in the Kabat database.

The **framework region** of an immunoglobulin heavy and light chain variable domain has a particular 3 dimensional conformation characterised by the presence of an immunoglobulin fold. Certain amino acid residues present in the variable domain are responsible for maintaining this characteristic immunoglobulin domain core structure. These residues are known as framework residues and tend to be highly conserved. The framework supports the CDRs of an antibody.

**CDR (complementarity determining region)** of an immunoglobulin molecule heavy and light chain variable domain describes those amino acid residues which are not framework region residues and which are contained within the hypervariable loops of the variable regions. These hypervariable loops are directly involved with the interaction of the immunoglobulin with the ligand. Residues within these loops tend to show less degree of conservation than those in the framework region.

**Intracellular means** inside a cell, and the present invention is directed to those immunoglobulins which will bind to ligands/targets selectively within a cell. The cell may be any cell, prokaryotic or eukaryotic, and is preferably selected from the group consisting of a bacterial cell, a yeast cell and a higher eukaryote cell. Most preferred are yeast cells and mammalian cells. As used herein, therefore, "intracellular" immunoglobulins and targets or ligands are immunoglobulins and targets/ligands which are present within a cell. In addition the term 'Intracellular' refers to environments which resemble or mimic an intracellular environment. Thus, "intracellular" may refer to an environment which is not within the cell, but is *in vitro.* For example, the method of the invention may be performed in an *in vitro* transcription and/or translation system, which may be obtained commercially, or derived from natural systems.

**Consensus sequence of V_{H} and V_{L} chains** in the context of the present invention refers to the consensus sequences of those V_{H} and V_{L} chains from immunoglobulin molecules which can bind selectively to a ligand in an intracellular environment. The residue which is most common in any one given position, when the sequences of those immunoglobulins which can bind intracellularly are compared is chosen as the consensus residue for that position. The consensus sequence is generated by comparing the residues for all the intracellularly binding immunoglobulins, at each position in turn, and then collating the data.

**Specific (antibody) binding** in the context of the present invention, means that the interaction between the antibody and the ligand are selective, that is, in the event that a number of molecules are presented to the antibody, the latter will only bind to one or a few of those molecules presented. Advantageously, the antibody-ligand interaction will be of high affinity. The interaction between immunoglobulin and ligand will be mediated by non-covalent interactions such as hydrogen bonding and Van der Waals forces.

A repertoire in the context of the present invention refers to a set of molecules generated by random, semi-random or directed variation of one or more template molecules, at the nucleic acid level, in order to provide a multiplicity of binding specificities. In this case the template molecule is one or more of the VH and/or VL domain sequences herein described. Methods for generating repertoires are well characterised in the art.

### a) Single Domain Immunoglobulins

Single domain immunoglobulin molecules are, typically, a single target-binding domain of an immunoglobulin divorced from other domains, especially other target-binding domains. For example, single domain immunoglobulins may be single domain antibodies, known in the art as DAbs, which consist of the heavy chain variable domain (V_{H}) or light chain variable domain (V_{L}) of an antibody.

The single domain immunoglobulins according to the invention are especially indicated for diagnostic and therapeutic applications. Accordingly, they may be altered antibodies comprising an effector protein such as a toxin or a label. Especially preferred are labels which allow the imaging of the distribution of the antibody in vivo. Such labels may be radioactive labels or radioopaque labels, such as metal particles, which are readily visualisable within the body of a patient. Moreover, they may be fluorescent labels or other labels which are visualisable on tissue samples removed from patients. Effector groups may be added prior to the selection of the antibodies by the method of the present invention, or afterwards.

Antibodies from which single domains may be derived may themselves be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product.

Multiplication of hybridoma cells or mammalian host cells *in vitro* is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

In vitro production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells in vivo. For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein. (1975) Nature 256:495-497; US 4,376,110; Harlow and Lane, Antibodies: a Laboratory Manual, (1988) Cold spring Harbor. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired target by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-)affinity chromatography, e.g affinity chromatography with the target molecule or with Protein-A.

Antibodies generated according to the foregoing procedures may be cloned by isolation of nucleic acid from cells, according to standard procedures. Usefully, nucleic acids variable domains of the antibodies may be isolated and used to construct antibody single domains, such as V_{H} or V_{L} domains.

The invention therefore preferably employs recombinant nucleic acids comprising an insert coding for a heavy chain variable domain or a light chain variable domain of antibodies. By definition such nucleic acids comprise coding single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain or a light chain variable domain of antibodies can be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain or a light chain variable domain of the above-mentioned antibodies in which one or more amino acids are deleted or exchanged with one or more other amino acids. Preferably said modification(s) are outside the CDRs of the heavy chain variable domain or of the light chain variable domain. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain or a light chain variable domain.

The term mutant is intended to include a DNA mutant obtained by *in vitro* or *in vivo* mutagenesis of DNA according to methods known in the art.

Recombinant DNA technology may be used to improve the antibodies of the invention. Thus, chimeric antibodies may be constructed in order to decrease the immunogenicity thereof in diagnostic or therapeutic applications. Moreover, immunogenicity may be minimised by humanising the antibodies by CDR grafting [as reviewed in European Patent Application 0 239 400 (Winter)] and, optionally, framework modification [as reviewed in international patent application WO 90/07861 (Protein Design Labs)].

The invention therefore also employs recombinant nucleic acids comprising an insert coding for a heavy chain variable domain of an antibody fused to a human constant domain γ, for example γ1 γ2, γ3 or γ4, preferably γ1 or γ4. Likewise the invention concerns recombinant DNAs comprising an insert coding for a light chain variable domain of an antibody fused to a human constant domain κ or λ, preferably κ.

More preferably, the invention employs CDR-grafted antibodies, which are preferably CDR-grafted light chain or heavy chain variable domains only.

Antibodies may moreover be generated by mutagenesis of antibody genes to produce artificial repertoires of antibodies. This technique allows the preparation of antibody libraries, as discussed further below; antibody libraries are also available commercially. Hence, the present invention advantageously employs artificial repertoires of single domain immunoglobulins, preferably artificial V_{H} repertoires.

Single domain immunoglobulins may be prepared by any suitable technique. The preparation of single domain antibodies is described in detail in Ward et al., (1989) Nature 341: 544-546 and in European Patent Application 0 368 684 (Medical Research Council).

### b) Targets

Targets are chosen according to the use to which it is intended to put the intracellular single domain immunoglobulin selected by the method of the present invention. Thus, where it is desired to select an immunoglobulin capable of binding to a defined cellular component, such as a polypeptide, a subcellular structure or an intracellular pathogen, the whole of said component or an epitope derived therefrom may be used as a target

Potential targets include polypeptides, particularly nascent polypeptides or intracellular polypeptide precursors, which are present in the cell. Advantageously, the target is a mutant polypeptide, such as a polypeptide generated through genetic mutation, including point mutations, deletions and chromosomal translocations. Such polypeptides are frequently involved in tumourigenesis. Examples include the gene product produced by the spliced BCR-ABL genes and point mutants of the *Ras* oncogene. The invention is moreover applicable to all mutated oncogene products, all chromosomal translocated oncogene products (especially fusion proteins), aberrant proteins in expressed in disease, and viral or bacterial specific proteins expressed as a result of infection.

The target may alternatively be an RNA molecule, for example a precursor RNA or a mutant RNA species generated by genetic mutation or otherwise.

The target may be inserted into the cell, for example as described below, or may be endogenous to the cell. Where the target is endogenous, generation of the signal is dependent on the attachment of a signalling molecule to the target within the cell, or on the target itself being capable of functioning as one half of the signal-generating agent.

### c) Libraries and selection systems

Immunoglobulins for use in the invention may be isolated from libraries comprising artificial repertoires of immunoglobulin polypeptides. Optionally, the immunoglobulins may be preselected by screening against the desired target, such that the method of the invention is performed with immunoglobulins which substantially all are specific for the intended target.

Any library selection system may be used in conjunction with the invention. Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences that encoding them) for the *in vitro* selection and amplification of specific antibody fragments that bind a target antigen. The nucleotide sequences encoding the V_{H} and V_{L} regions are linked to gene fragments which encode leader signals that direct them to the periplasmic space of *E*. *coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88; 10134; Hoogenboom et al. (1991) Nucleic Acids Res., 19: 4133; Chang et al. (1991) J. Immunol., 147; 3610; Breitling et al. (1991) Gene, 104: 147; Marks et al. (1991) J. Mol. Biol., 222: 581**;** Barbas et al, (1992) Proc. Natl. Acad Sci. USA, 89: 4457; Hawkins and Winter (1992) J. Immunol., 22: 867; Marks et al., 1992, J. Biol. Chem., 267: 16007; Lerner et al. (1992) Science. 258: 1313.

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson et al. (1991) Nature, 352: 624; Marks *et al.* (1991) supra; Chiswell et al. (1992) Trends Biotech., 10: 80; Marks et al. (1992) J. Biol. Chem., 267). Various embodiments of scFv libraries displayed on bacteriophage coat proteins have been described. Refinements of phage display approaches are also known, for example as described in WO96/06213 and WO92/01047 (Medical Research Council *et al.*) and WO97/08320 (Morphosys). Methods suitable for the selection of scFv libraries may be applied to the preselection of single domains (DAbs) for use in the present invention.

Alternative library selection technologies include bacteriophage lambda expression systems, which may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87**;** Mullinax et al. (1990) Proc. Natl. Acad. Sci. U.S.A., 87: 8095; Persson et al. (1991) Proc. Natl. Acad. Sci. U.S.A., 88: 2432) and are of use in the invention. Whilst such expression systems can be used to screening up to 10⁶ different members of a library, they are not really suited to screening of larger numbers (greater than 10⁶ members). Other screening systems rely, for example, on direct chemical synthesis of library members. One early method involves the synthesis of peptides on a set of pins or rods, such as described in WO84/03564. A similar method involving peptide synthesis on beads, which forms a peptide library in which each bead is an individual library member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121.

Another chemical synthesis method involves the synthesis of arrays of peptides (or peptidomimetics) on a surface in a manner that places each distinct library member (e.g., unique peptide sequence) at a discrete, predefined location in the array. The identity of each library member is determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092; Fodor et al. (1991) Science, 251: 767; Dower and Fodor (1991) Ann. Rep. Med. Chem., 26: 271.

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bath (1990) Nucleic Acids Res., 18: 3203; Beaudry and Joyce (1992) Science, 257: 635; WO92/05258 and WO92/14843) In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection.

An alternative to the use of phage or other cloned libraries is to use nucleic acid, preferably RNA, derived from the spleen of an animal which has been immunised with the selected target. RNA thus obtained represents a natural library of immunoglobulins. Isolation of V-region mRNA permits single domain antibody fragments, such as V_{H} or V_{L}, to be expressed intracellularly in accordance with the invention.

Briefly, RNA is isolated from the spleen of an immunised animal and PCR primers used to amplify V_{H} and V_{L} cDNA selectively from the RNA pool. PCR primer sequences are based on published V_{H} and V_{L} sequences and are available commercially in kit form.

### d) Delivery of Immunoglobulins and Targets to Cells

The present invention provides an assay for intracellular antibodies which is conducted essentially intracellularly, or in conditions which mimic the intracellular environment, preferably the cytoplasmic environment. Moreover, the immunoglobulins according to the invention are useful inside the cytoplasm or nucleus of a cell. Accordingly, the invention provides methods for delivery of nucleic acid constructs encoding immunoglobulins and/or targets, and methods for delivering polypeptides, to the interior of a cell.

In order to introduce immunoglobulins and target molecules into an intracellular environment, cells are advantageously transfected with nucleic acids which encode the immunoglobulins and/or their targets.

Nucleic acids encoding immunoglobulins and/or targets can be incorporated into vectors for expression. As used herein, vector (or plasmid) refers to discrete elements that are used to introduce heterologous DNA into cells for expression thereof. Selection and use of such vehicles are well within the skill of the artisan. Many vectors are available, and selection of appropriate vector will depend on the intended use of the vector, the size of the nucleic acid to be inserted into the vector, and the host cell to be transformed with the vector. Each vector contains various components depending on its function and the host cell for which it is compatible. The vector components generally include, but are not limited to, one or more of the following: an origin of replication, one or more marker genes, an enhancer element, a promoter, a transcription termination sequence and a signal sequence.

Moreover, nucleic acids encoding the immunoglobulins and/or targets according to the invention may be incorporated into cloning vectors, for general manipulation and nucleic acid amplification purposes.

Both expression and cloning vectors generally contain nucleic acid sequence that enable the vector to replicate in one or more selected host cells. Typically in cloning vectors, this sequence is one that enables the vector to replicate independently of the host chromosomal DNA, and includes origins of replication or autonomously replicating sequences. Such sequences are well known for a variety of bacteria, yeast and viruses. The origin of replication from the plasmid pBR322 is suitable for most Gram-negative bacteria, the 2m plasmid origin is suitable for yeast, and various viral origins (e.g. SV 40, polyoma, adenovirus) are useful for cloning vectors in mammalian cells. Generally, the origin of replication component is not needed for mammalian expression vectors unless these are used in mammalian cells competent for high level DNA replication, such as COS cells.

Most expression vectors are shuttle vectors, i.e. they are capable of replication in at least one class of organisms but can be transfected into another class of organisms for expression. For example, a vector is cloned in E. coli and then the same vector is transfected into yeast or mammalian cells even though it is not capable of replicating independently of the host cell chromosome. DNA may also be replicated by insertion into the host genome. However, the recovery of genomic DNA is more complex than that of exogenously replicated vector because restriction enzyme digestion is required to excise the nucleic acid. DNA can be amplified by PCR and be directly transfected into the host cells without any replication component.

Advantageously, an expression and cloning vector may contain a selection gene also referred to as selectable marker. This gene encodes a protein necessary for the survival or growth of transformed host cells grown in a selective culture medium. Host cells not transformed with the vector containing the selection gene will not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available from complex media.

As to a selective gene marker appropriate for yeast, any marker gene can be used which facilitates the selection for transformants due to the phenotypic expression of the marker gene. Suitable markers for yeast are, for example, those conferring resistance to antibiotics G418, hygromycin or bleomycin, or provide for prototrophy in an auxotrophic yeast mutant, for example the URA3, LEU2, LYS2, TRP1, or HIS3 gene.

Since the replication of vectors is conveniently done in *E. coli,* an *E. coli* genetic marker and an E. coli origin of replication are advantageously included. These can be obtained from E. coli plasmids, such as pBR322, Bluescript© vector or a pUC plasmid, e.g. pUC18 or pUC19, which contain both an *E. coli* replication origin and an *E. coli* genetic marker conferring resistance to antibiotics, such as ampicillin.

Suitable selectable markers for mammalian cells are those that enable the identification of cells expressing the desired nucleic acid, such as dihydrofolate reductase (DHFR, methotrexate resistance), thymidine kinase, or genes conferring resistance to G418 or hygromycin. The mammalian cell transformants are placed under selection pressure which only those transformants which have taken up and are expressing the marker are uniquely adapted to survive. In the case of a DHFR or glutamine synthase (GS) marker, selection pressure can be imposed by culturing the transformants under conditions in which the pressure is progressively increased, thereby leading to amplification (at its chromosomal integration site) of both the selection gene and the linked nucleic acid. Amplification is the process by which genes in greater demand for the production of a protein critical for growth, together with closely associated genes which may encode a desired protein, are reiterated in tandem within the chromosomes of recombinant cells. Increased quantities of desired protein are usually synthesised from thus amplified DNA.

Expression and cloning vectors usually contain a promoter that is recognised by the host organism and is operably linked to the desired nucleic acid. Such a promoter may be inducible or constitutive. The promoters are operably linked to the nucleic acid by removing the promoter from the source DNA and inserting the isolated promoter sequence into the vector. Both the native promoter sequence and many heterologous promoters may be used to direct amplification and/or expression of nucleic acid encoding the immunoglobulin or target molecule. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Their nucleotide sequences have been published, thereby enabling the skilled worker operably to ligate them a desired nucleic acid, using linkers or adaptors to supply any required restriction sites. Promoters for use in bacterial systems will also generally contain a Shine-Delgarno sequence operably linked to the nucleic acid.

Preferred expression vectors are bacterial expression vectors which comprise a promoter of a bacteriophage such as phagex or T7 which is capable of functioning in the bacteria. In one of the most widely used expression systems, the nucleic acid encoding the fusion protein may be transcribed from the vector by T7 RNA polymerase (Studier et al, Methods in Enzymol. 185; 60-89, 1990). In the *E. coli* BL21(DE3) host strain, used in conjunction with pET vectors, the T7 RNA polymerase is produced from the λ-lysogen DE3 in the host bacterium, and its expression is under the control of the IPTG inducible lac UV5 promoter. This system has been employed successfully for over-production of many proteins. Alternatively the polymerase gene may be introduced on a lambda phage by infection with an int- phage such as the CE6 phage which is commercially available (Novagen, Madison, USA). other vectors include vectors containing the lambda PL promoter such as PLEX (Invitrogen, NL), vectors containing the trc promoters such as pTrcHisXpressTm (Invitrogen) or pTrc99 (Pharmacia Biotech, SE), or vectors containing the tac promoter such as pKK223-3 (Pharmacia Biotech) or PMAL (new England Biolabs, MA, USA).

Suitable promoting sequences for use with yeast hosts may be regulated or constitutive and are preferably derived from a highly expressed yeast gene, especially a Saccharomyces cerevisiae gene. Thus, the promoter of the TRP1 gene, the ADHI or ADHII gene, the acid phosphatase (PH05) gene, a promoter of the yeast mating pheromone genes coding for the a- or α-factor or a promoter derived from a gene encoding a glycolytic enzyme such as the promoter of the enolase, glyceraldehyde-3-phosphate dehydrogenase (GAP), 3-phospho glycerate kinase (PGK), hexokinase, pyruvate decarboxylase, phosphofructokinase, glucose-6-phosphate isomerase, 3-phosphoglycerate mutase, pyruvate kinase, triose phosphate isomerase, phosphoglucose isomerase or glucokinase genes, the *S. cerevisiae* GAL 4 gene, the *S. pombe* nmt 1 gene or a promoter from the TATA binding protein (TBP) gene can be used. Furthermore, it is possible to use hybrid promoters comprising upstream activation sequences (UAS) of one yeast gene and downstream promoter elements including a functional TATA box of another yeast gene, for example a hybrid promoter including the UAS(s) of the yeast PH05 gene and downstream promoter elements including a functional TATA box of the yeast GAP gene (PH05-GAP hybrid promoter). A suitable constitutive PHO5 promoter is e.g. a shortened acid phosphatase PH05 promoter devoid of the upstream regulatory elements (UAS) such as the PH05 (-173) promoter element starting at nucleotide -173 and ending at nucleotide -9 of the PH05 gene.

Gene transcription from vectors in mammalian hosts may be controlled by promoters derived from the genomes of viruses such as polyoma virus, adenovirus, fowlpox virus, bovine papilloma virus, avian sarcoma virus, cytomegalovirus (CMV), a retrovirus and Simian Virus 40 (SV40), from heterologous mammalian promoters such as the actin promoter or a very strong promoter, e.g. a ribosomal protein promoter, and from promoters normally associated with immunoglobulin sequences.

Transcription of a nucleic acid by higher eukaryotes may be increased by inserting an enhancer sequence into the vector. Enhancers are relatively orientation and position independent. Many enhancer sequences are known from mammalian genes (e.g. elastase and globin). However, typically one will employ an enhancer from a eukaryotic cell virus. Examples include the SV40 enhancer on the late side of the replication origin (bp 100-270) and the CMV early promoter enhancer. The enhancer may be spliced into the vector at a position 5' or 3' to the desired nucleic acid, but is preferably located at a site 5' from the promoter.

Advantageously, a eukaryotic expression vector may comprise a locus control region (LCR). LCRs are capable of directing high-level integration site independent expression of transgenes integrated into host cell chromatin, which is of importance especially where the gene is to be expressed in the context of a permanently-transfected eukaryotic cell line in which chromosomal integration of the vector has occurred.

Eukaryotic expression vectors will also contain sequences necessary for the termination of transcription and for stabilising the mRNA. Such sequences are commonly available from the 5' and 3' untranslated regions of eukaryotic or viral DNAs or cDNAs. These regions contain nucleotide segments transcribed as polyadenylated fragments in the untranslated portion of the mRNA encoding the immunoglobulin or the target.

Particularly useful for practising the present invention are expression vectors that provide for the transient expression of nucleic acids in mammalian cells. Transient expression usually involves the use of an expression vector that is able to replicate efficiently in a host cell, such that the host cell accumulates many copies of the expression vector, and, in turn, synthesises high levels of the desired gene product.

Construction of vectors according to the invention may employ conventional ligation techniques. Isolated plasmids or DNA fragments are cleaved, tailored, and religated in the form desired to generate the plasmids required. If desired, analysis to confirm correct sequences in the constructed plasmids is performed in a known fashion. Suitable methods for constructing expression vectors, preparing in vitro transcripts, introducing DNA into host cells, and performing analyses for assessing gene product expression and function are known to those skilled in the art. Gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe which may be based on a sequence provided herein. Those skilled in the art will readily envisage how these methods may be modified, if desired.

Immunoglobulins and/or targets may be directly introduced to the cell by microinjection, or delivery using vesicles such as liposomes which are capable of fusing with the cell membrane. Viral fusogenic peptides are advantageously used to promote membrane fusion and delivery to the cytoplasm of the cell.

Preferably, the immunoglobulin is fused or conjugated to a domain or sequence from such a protein responsible for translocational activity. Preferred translocation domains and sequences include domains and sequences from the HIV-1-trans-activating protein (Tat), *Drosophila* Antennapedia homeodomain protein, the TLM peptide, anti-DNA antibody peptide technology and the herpes simplex-1 virus VP22 protein. By this means, the immunoglobulin is able to enter the cell or its nucleus when introduced in the vicinity of the cell.

Exogenously added HIV-1-trans-activating protein (Tat) can translocate through the plasma membrane and to reach the nucleus to transactivate the viral genome. Translocational activity has been identified in amino acids 37-72 (Fawell et al., 1994, Proc. Natl. Acad Sci. U. S. A. 91, 664-668), 37-62 (Anderson et al., 1993, Biochem. Biophys. Res. Commun. 194, 876-884) and 49-58 (having the basic sequence RKKRRQRRR) of HIV-Tat. Vives et al. (1997), J Biol Chem 272, 16010-7 identified a sequence consisting of amino acids 48-60 (CGRKKRRQRRRPPQC), which appears to be important for translocation, nuclear localisation and trans-activation of cellular genes. Intraperitoneal injection of a fusion protein consisting of β-galactosidase and a HIV-TAT protein transduction domain results in delivery of the biologically active fusion protein to all tissues in mice (Schwarze et al., 1999, Science 285,1569-72)

The third helix of the *Drosophila* Antennapedia homeodomain protein has also been shown to possess similar properties (reviewed in Prochiantz, A., 1999, Ann N YAcad Sci, 886, 172-9). The domain responsible for translocation in Antennapedia has been localised to a 16 amino acid long peptide rich in basic amino acids having the sequence RQIKIWFQNRRMKWKK (Derossi, et al., 1994, J Biol Chem, 269, 10444-50). This peptide has been used to direct biologically active substances to the cytoplasm and nucleus of cells in culture (Theodore, et al., 1995, J. Neurosci 15, 7158-7167). Cell internalisation of the third helix of the Antennapedia homeodomain appears to be receptor-independent, and it has been suggested that the translocation process involves direct interactions with membrane phospholipids (Derossi et al., 1996, J Biol Chem, 271, 18188-93).

The VP22 tegument protein of herpes simplex virus is capable of intercellular transport, in which VP22 protein expressed in a subpopulation of cells spreads to other cells in the population (Elliot and O'Hare, 1997, Cell 88, 223-33). Fusion proteins consisting of GFP (Elliott and O'Hare, 1999, Gene Ther 6, 149-51), thymidine kinase protein (Dilber et al., 1999, Gene Ther 6, 12-21) or p53 (Phelan et al., 1998, Nat Biotechnol 16, 440-3) with VP22 have been targeted to cells in this manner.

The TLM peptide is derived from the Pre-S2 polypeptide of HBV. See Oess S, Hildt E Gene Ther 2000 May 7:750-8. Anti-DNA antibody peptide technology is described in Alexandre Avrameas et al., PNAS val 95, pp 5601-5606, May 1998; Thérèse Ternynck et al., Journal of Autoimmunity (1998) 11, 511-521; and Bioconjugate Chemistry (1999), vol 10 Number 1, pp 87-93.

Particular domains or sequences from proteins capable of translocation through the nuclear and/or plasma membranes may be identified by mutagenesis or deletion studies. Alternatively, synthetic or expressed peptides having candidate sequences may be linked to reporters and translocation assayed. For example, synthetic peptides may be conjugated to fluoroscein and translocation monitored by fluorescence microscopy by methods described in Vives et al. (1997), J Biol Chem 272, 16010-7. Alternatively, green fluorescent protein may be used as a reporter (Phelan et al., 1998, Nat Biotechnol 16, 440-3).

Any of the domains or sequences or as set out above or identified as having translocational activity may be used to direct the immunoglobulins into the cytoplasm or nucleus of a cell. The Antennapedia peptide described above, also know as penetratin, is preferred, as is HIV Tat. Translocation peptides may be fused N-terminal or C-terminal to single domain immunoglobulins according to the invention. N-terminal fusion is preferred.

### e) Generation of a Signal

In the method of the present invention, a signal is advantageously generated by the interaction of two molecules, brought together by the binding of the immunoglobulin to the target. The signal generated will thus be dependent on the nature of the molecules used in the method of the invention.

In a first embodiment, the signal-generation molecules may be fluorophores. Particularly preferred are fluorescent molecules which participate in energy transfer (FRET).

FRET is detectable when two fluorescent labels which fluoresce at different frequencies are sufficiently close to each other that energy is able to be transferred from one label to the other. FRET is widely known in the art (for a review, see Matyus, 1992, J. Photochem. Photobiol. B: Biol., 12: 323-337, reference). FRET is a radiationless process in which energy is transferred from an excited donor molecule to an acceptor molecule; the efficiency of this transfer is dependent upon the distance between the donor an acceptor molecules, as described bellow Since the rate of energy transfer is inversely proportional to the sixth power of the distance between the donor and acceptor, the energy transfer efficiency is extremely sensitive to distance changes. Energy transfer is said to occur with detectable efficiency in the 1-10 nm distance range, but is typically 4-6 nm for favourable pairs of donor and acceptor.

Radiationless energy transfer is based on the biophysical properties of fluorophores. These principles are reviewed elsewhere (Lakowicz, 1983, Principles of Fluorescence Spectroscopy, Plenum Press, New York, Jovin and Jovin, 1989, Cell Structure and Function by Microspectrofluorometry, eds. E. Kohen and J.G. Hirschberg, Academic Press). Briefly, a fluorophore absorbs light energy at a characteristic wavelength. This wavelength is also known as the excitation wavelength. The energy absorbed by a fluorochrome is subsequently released through various pathways, one being emission of photons to produce fluorescence. The wavelength of light being emitted is known as the emission wavelength and is an inherent characteristic of a particular fluorophore. Radiationless energy transfer is the quantum-mechanical process by which the energy of the excited state of one fluorophore is transferred without actual photon emission to a second fluorophore. That energy may then be subsequently released at the emission wavelength of the second fluorophore. The first fluorophore is generally termed the donor (D) and has an excited state of higher energy than that of the second fluorophore, termed the acceptor (A). The essential features of the process are that the emission spectrum of the donor overlap with the excitation spectrum of the acceptor, and that the donor and acceptor be sufficiently close. The distance over which radiationless energy transfer is effective depends on many factors including the fluorescence quantum efficiency of the donor, the extinction coefficient of the acceptor, the degree of overlap of their respective spectra, the refractive index of the medium, and the relative orientation of the transition moments of the two fluorophores. In addition to having an optimum emission range overlapping the excitation wavelength of the other fluorophore, the distance between D and A must be sufficiently small to allow the radiationless transfer of energy between the fluorophores.

In a FRET assay, the fluorescent molecules are chosen such that the excitation spectrum of one of the molecules (the acceptor molecule) overlaps with the emission spectrum of the excited fluorescent molecule (the donor molecule). The donor molecule is excited by light of appropriate intensity within the donor's excitation spectrum. The donor then emits some of the absorbed energy as fluorescent light and dissipates some of the energy by FRET to the acceptor fluorescent molecule. The fluorescent energy it produces is quenched by the acceptor fluorescent molecule. FRET can be manifested as a reduction in the intensity of the fluorescent signal from the donor, reduction in the lifetime of its excited state, and re-emission of fluorescent light at the longer wavelengths (lower energies) characteristic of the acceptor. When the donor and acceptor molecules become spatially separated, FRET is diminished or eliminated.

Suitable fluorophores are known in the art, and include chemical fluorophores and fluorescent polypeptides, such as GFP and mutants thereof which fluoresce with different wavelengths or intensities (see WO 97/28261). Chemical fluorophores may be attached to immunoglobulin or target molecules by incorporating binding sites therefor into the immunoglobulin or target molecule during the synthesis thereof.

Preferably, however, the fluorophore is a fluorescent protein, which is advantageously GFP or a mutant thereof. GFP and its mutants may be synthesised together with the immunoglobulin or target molecule by expression therewith as a fusion polypeptide, according to methods well known in the art. For example, a transcription unit may be constructed as an in-frame fusion of the desired GFP and the immunoglobulin or target, and inserted into a vector as described above, using conventional PCR cloning and ligation techniques.

In a second embodiment, the immunoglobulin and target polypeptides are associated with molecules which give rise to a biological signal. Preferred are polypeptide molecules, which advantageously interact to form a transcription factor, or another regulatory molecule, which modulates gene expression within the cell.

Exemplary transcription factor molecules have been described in the literature, for example by Fields & Song, (1989) Nature 340:245-246. In a preferred embodiment, the immunoglobulin molecule is expressed as fusion protein with the activation domain of the HSV1 VP16 molecule. This transcription factor domain is capable of upregulating gene transcription from a promoter to which it is bound through a DNA binding activity. The latter is provided by them DNA-binding domain of the *E. coli* LexA polypeptide, which is expressed as a fusion protein with the target polypeptide. Other DNA binding domains (DBDs), such as the Gal4 DBD, may also be used, as may other transcription activation domains derived from a variety of transcription factors. Combinations of LexA, Ga14 and VP16 are commonly used. The operation of two-hybrid assay systems is described in detail in the following: Golemis, E.A. and Serebriiskii, I. Recent developments in two hybrid technology. In the 3rd sedition of Molecular Cloning: a Laboratory Manual, ed. J. Sambrook. Cold Spring Harbor Laboratory Press, 2000 (the former Maniatis Cloning manual). Includes both review and a protocol; "Methods in Molecular Biology: Two Hybrid Systems, Methods, and Protocols", ed. P.MacDonald. Humana Press; Serebriiskii, I., G.Toby, R.L. Finley, and E.A.Golemis. Genomic analysis utilising the yeast two-hybrid system. In: "Methods in Molecular Biology: Genomic Protocols", ed. M. Starkey. Humana Press; Fashena, S. J., Secebriiskii, I., and Golemis, E.A. LexA based two hybrid systems. In "Methods in Enzymology: Chimeric Genes and Proteins", ed. J.Abelson, M.Simon, S.Emr, J.Thorner. Academic Press; Serebriiskii, I., Mitina, O., Chernoff, J. , and E.A.Golemis. Use of a two-hybrid dual bait system to discriminate specificity of protein interactions in small GTPases. In "Methods in Enzymology: Ras Regulators and Effectors", ed. C.J. Der. Academic Press.

The biological signal may be any detectable signal, such as the induction of the expression of a detectable gene product. Examples of detectable gene products include bioluminescent polypeptides, such as luciferase and GFP, polypeptides detectable by specific assays, such as β-galactosidase and CAT, and polypeptides which modulate the growth characteristics of the host cell, such as enzymes required for metabolism such as HIS3, or antibiotic resistance genes such as G418. In a preferred aspect of the invention, the signal is detectable at the cell surface. For example, the signal may be a luminescent or fluorescent signal, which is detectable from outside the cell and allows cell sorting by FACS or other optical sorting techniques. Alternatively, the signal may comprise the expression of a cell surface marker, such as a CD molecule, for example CD4 or CD8, which may itself be labelled, for example with a fluorescent group, or may be detectable using a labelled antibody.

In this embodiment, the invention permits the screening of entire antibody libraries, such as phage libraries, without prior application of phage display to isolate the antibodies which bind to the desired antigen. Use of optical sorting, such as FACS, enables an entire library to be panned and selects for antibodies which are capable of functioning intracellularly and bind the desired target.

In summary, therefore, the invention is related to a method for determining the ability of a single domain entity to bind to a target in an intracellular environment, comprising the steps of providing a first molecule and a second molecule, wherein stable interaction of the first and second molecules leads to the generation of a signal; providing an entity which is associated with the first molecule; providing a target which is associated with the second molecule, such that association of the entity and the target leads to stable interaction of the first and second molecules and generation of the signal; and assessing the intracellular interaction between the entity and the target by monitoring the signal. In preferred embodiments, the entity is a single domain immunoglobulin, preferably a single domain antibody, and the target is an antigen.

The invention is further described, for the purposes of illustration only, in the following examples.

### Examples

Reagents that can be rapidly isolated and interfere with function are key components of the functional genomics arm of genome projects, like the Human Genome Project ⁶. Intrabodies are also attractive reagents for intracellular targets in disease and different approaches have been devised to overcome the limited effectiveness of scFv ^{4,5,7,8}. Intracellular antibody capture (IAC) technology has helped to define a scaffold of imunoglobulin V-region residues which are particularly advantageous for in cell function ^{4,9} A numerical limitation of using scFv intrabodies is the combinatorial effect of heavy and light chains and the subsequent diversity required for initial screening for antigen-specific intrabodies. The smallest imunoglobulin-based recognition units so far defined are single variable domaines ¹⁰ with the potential advantage that the overall complexity for screening will be lower than scFv ^{11,12}. In our previous study ⁹, anti-HRAS scFv intrabodies were isolated by IAC ^{4,5} and we have now tested individual domain (i.e. VH or VL domains) binding antigen *in vivo*. Antibody fragments were tested in a luciferase reporter assay ⁹ which comprised transfecting COS7 cells with a minimal luciferase reporter clone together with vectors encoding either RAS linked to the Gal4 DNA binding domain (DBD) or intrabody fragment linked to the VP16 transcriptional activation domain (AD). The antibody expressing clones are illustrated in Figure 1 and levels of expression of the intrabodies compared, showing similar protein levels produced in each case. The levels of luciferase activation following binding of DBD-antigen by the intrabody-VP16 fusion protein were compared (Figure 1). Significantly, the best luciferase activation was achieved with the anti-RAS VH single domain formats. For instance, the VH segment from the anti-RAS scFv33 ⁹ (Figure 1, 33VH) stimulates the reporter activity about 5 times more that the parental scFv clone (Figure 1, 33). The anti-RAS VL single domain did not activate at all (33VL). In addition, mutation of the cysteine codons (involved in the intra-domain disulphide bond) has no substantial effect on *in vivo* function (clones I21R33VH-C22S and I21R33VH-C92S). Thus binding of the anti-RAS scFv33 to antigen can occur through the VH domain alone, in turn suggesting that single domains can be mediators of intrabody function.

These data suggested that the single domain intrabody format (IDabs), coupled with the previously described optimal intrabody consensus framework ^{4,9} could be used for production of sufficiently diverse intrabody libraries for direct *in vivo* isolation of antigen-specific IDabs. We have generated such libraries for *in vivo* screening in the yeast antigen-antibody interaction assay ^{4,5}. Two pooled libraries have been made by cloning diversified VH domains into a yeast vector to encode Dab-VP 16 fusion proteins (Figure 2A). Each Dab library was screened with three different antigens, RAS, p53 and ATF-2 (a member of the CREB/ATF family of transcriptional regulators) to ascertain their general utility. Yeast cells with *HIS3* and *lacZ* reporter genes, were transfected with antigen bait clones expressing the antigen fused to the LexA DBD and transfected with the IDab libraries. More than 100 IDab clones were isolated with each antigen (except Dab library 1 with the p53 bait which yielded only 16 clones) (Figure 2A). Ten clones giving most rapid colour development for respective antigen were selected for further study. Among the selected clones, some identical IDabs were found with same antigen (for example, anti-p53 clones #102, #103, and #109). In addition, surveying all the clones showed that clones #1, #14 (from RAS selection), and #105 plus #107 (from p53 selection) had identical sequence suggesting that these clones bind with LexA DBD. This was assessed by re-assaying each IDab clone with each bait to determine the specificity against their respective antigen (Figure 2B).

The efficacy of the IDabs was tested in mammalian cells using three transcriptional transactivation assays (Figure 3). IDabs were tested in a COS7 luciferase reporter assay ⁹. Each was cloned into a mammalian expression vector, pEF-VP16 ¹³, to express the IDab fused with the VP16AD. COS7 cells were co-transfected with the pEF-IDab-VP16 constructs and either the specific bait or a bait comprising Ga14DBD-LexA fusion (Figure 3A). Some clones gave a high stimulation in reporter activity, for instance anti-RAS clones #6 and #10 (Figure 3A, top left hand panel) and some only a moderate stimulation, for instance anti-RAS clone #3 (top right hand panel) or the anti-ATF2 clones #27 and #29 (Figure 3B). Interestingly, anti-RAS clone #3 has a long CDR3, compared to other anti-RAS IDab (Fig 2B), but only showed luciferase activation via with HRAS, not with K-RAS and N-RAS whereas the anti-RAS Dab clones #6, #7, #9, #10, #12, #13, #17 and #18 could bind the three forms of RAS (not shown). Conversely, clones #1, #2, #4, #11, #14, #16 and #19 showed significant increase in reporter activity against LexA antigen.

The anti-RAS IDabs was tested in Chinese hamster ovary cells (CHO) which carry either a chromosomal CD4 ¹⁴ or a green fluorescent protein (GFP) reporter (Figure 3 D and E, respectively). When a non-relevant, anti-β-gal scFvR4 ¹⁵, was co-expressed with the RAS bait in CHO-CD4, no reporter activation was observed, whereas around 18% of cells displayed CD4 expression when scFvR4 and a lacZ reporter were co-transfected (Figure 3D). The bait specificity was reversed when anti-RAS IDab33 (the original IDab converted from the anti-RAS scFv33 ⁹) IDab #6 or #10 (derived from the IDab libraries) were co-expressed with the baits, since activation was only observed with the RAS bait (Figure 3D). Parallel data were obtained when the CHO-GFP line was employed, in which the generation of GFP protein occurred in an antigen-specific manner (Figure 3E). These results indicate that the yeast Dab library screening approach can select IDabs with sufficiently good *in vivo* properties to facilitate binding in mammalian cells.

The *in vitro* affinitiies of four selected anti-RAS clones #3, #10, #12 were compared to the original Dab 33 using a biosensor. The Kd of scFv33 was 9.97 ± 8.82 nM (Table 1), consistent with our previous study ⁹. The mutated scFvI21R33VHI21VL (the framework of anti-RAS scFv33 is mutated to the I21 'consensus' VH but retains the I21 VL sequence) maintains the affinity of scFv33 (Kd of 18.19 ± 1.85 nM), consistent with the paramount importance of the VH-antigen interaction. Loss of affinity was observed when the VH of scFv33 was made into a Dab (Table 1; Kd of 90.13 ± 9.70 nM), being about one order of magnitude weaker than original scFv33. This suggests that VL domain of scFv plays a supportive role for recognition of antigen, although VH alone maintains specificity. The Kds of anti-RAS Dab clone #3, #10, and #12 were 182.98 ± 7.19 nM, 121.45 ± 46.6 nM, 26.65 ± 2.90 nM, respectively. Thus in the anti-RAS Dabs, including Dab33, there is no correlation between the *in vitro* affinity (which shows 'real' antibody-antigen interaction) and *in vivo* activity (which indicates the total antibody-antigen interaction involving several factors including *in vivo* solubility, stability, expression level).

The main purpose of intrabodies is to interfere with the function of proteins inside cells. The function of oncogenic RAS is mediated through constitutive signalling in tumours and this can be emulated by introducing mutant RAS (BRASG12V) into NIH3T3 cells, resulting in loss of contact inhibition and focus formation in confluent cell cultures. The effect of IDabs on transformation was assessed by transfecting NIH3T3 cells with HRASG12V in the presence or absence of intrabodies (Figure 4). When an expression clone encoding HRASG12V was transfected into NIH3T3 cells, transformed clones were detected (Figure 4A) whereas cells retained their contact inhibition when only vector was transfected. The transforming ability of the mutant RAS was unaffected when co-transfected with scFvI21 (an scFv which has no detectable RAS binding in mammalian assays ⁹) (Figure 4A and B). Conversely, an ablation of transformation occurred when HRASG12V was co-expressed with anti-RAS scFv (scFvI21R33VHI21VL in which the scFv comprises VH of anti-RAS scFv33 with VL of I21 ⁹), with only around 20% of the foci compared to HRASG12V alone (Figure 4B). Two anti-RAS IDabs were tested in this assay, Dab #6 and #10 chosen because of their stimulation in the mammalian reporter assays (Figure 3). These IDabs behaved like the anti-RAS scFv, showing a inhibitory effect on the transformation index. Anti-RAS Dab #6 and #10 reduced the transforming activity of oncogenic HRASG12V to around 10% of the positive control (Figure 4B), showing that the IDab selection procedure is able to generate reagents with sufficiently good *in vivo* properties to interfere with protein function.

The purpose of using intrabodies *in vivo* is to elicit a biological response through antigen binding, with potential application in functional genomic research and therapeutics. A robust, rapid and simple procedure to identify such intrabody fragments is required for these ends. Our expression strategy to screen diverse intrabody libraries *in vivo*, and directly isolate those which have *in vivo* activity ⁹, shows that single domains (in this case, VH alone but VL may possess the same property) can be more effective as intracellular reagents than scFv to generate sets of antigen-specific molecules. Single domain intrabodies are the smallest antibody-based recognition unit with potential for in cell therapeutic use at present. Application of the IAC technology ^{4,5,9} to single domain libraries has the immediate advantage of avoiding a phage antibody library screening step. An additional feature which increases the effectiveness of the IDab libraries is the use of our intracellular consensus VH framework sequence ^{4,9} as a suitable framework for specific intracellular library diversification, since these sequences display ideal properties for intracellular function, such as expression, solubility and functionality without conserved intra-domain disulphide bonds ⁹. A final key point about direct screening of IDab libraries is that no antigen purification is required to identify intrabodies, since only DNA sequence is needed to generate antigen baits *in vivo.* This has particular advantage for functional genomics applications where genome sequences generate novel open reading frames, for which functional data is sought. Thus IDabs are good candidates to serve as a lead tools for new therapeutics and functional genomic research.

### Methods

### Plasmids

Previuosly described plasmids are pM1-HRASG12V, pM1-LacZ, pEF-VP16-scFv33 (anti-RAS), pEF-VP16-scFvI21R33 (anti-RAS) ⁹ and pEF-VP16-scFvR4 (anti-lacZ) pG5-Luc ⁴, pBTM-ATF-2 ¹⁶ and pG5GFP-hyg (for CHO-GFP) ¹⁷. pRL-CMV was obtained from Promega Ltd.
For cloning mammalian expression clones pEF-33VH-VP16, pEF-I21R33VH-VP16, pEF-I21R33VHC22S-VP16, or pEF-I21R33VHC22S-VP16, the respective VH domain fragments were amplified from parental pEF-scFv-VP16 by PCR using oligonucleotides, EFFP, 5'-TCTCAAGCCTCAGACAGTGGTTC-3' and NotVHJR1' 5'-CATGATGATGTGCGGCCGCTCCACCTGAGGAGACGGTGACC-3' to introduce SfiI and NotI cloning sites and sub-cloned into SfiI-NotI site of pEF-VP16 ¹³. For cloning the mammalian expression clones pEF-33VL-VP16, pEF-I21R33VL-VP16, the respective VL domain fragments were amplified from parental pEF-scFv-VP16 by PCR using VLF1 5'-ATCATGCCATGGACATCGTGATGACCCAGTC-3' to introduce a NcoI cloning site and VP162R, 5'-CAACATGTCCAGATCGAA-3', and sub-cloned in frame into the NcoI-NotI site of pEF-VP16. The pBTM-p53wt and pM1-p53wt were created by sub-cloning the EcoRI-BamHI fragment from pGBT9-p53wt ¹⁸ into pBTM116 ¹⁹ or pM1 vectors ²⁰. The pEF-Dab-VP16 were made by cloning the respective SfiI-NotI fragments of isolated pVP16*-Dab (see below) into pEF-VP16. The baits pM1-ATF-2 was made by sub-cloning the SmaI-BamHI fragment from pBTM-ATF-2 ¹⁶ into the pM1 vector ²⁰. The pM1-LexA DBD clone was made by PCR amplifying the LexA fragment from the pBTM116 vector using BLEXAF2, 5'-CGCGGATCCTGAAAGCGTTAACGGCCAGG-3' and BAMLEXAR, 5'-CGCGGATCCAGCCAGTCGCCGTTGC-3', and cloned in frame intoBamHI site of pM1 vector.

For periplasmic expression, the pHEN2-scFv or Dab vectors were made by cloning the respective SfiI-NotI fragments of pEF-scFv-VP16 or pEF-Dab-VP16 into pHEN2 phagemid (see www.mrc-cpe.cam.ac.uk for map). The pZIPneoSV(X)-HRASG12V was made by cloning the coding sequence of HRASG12V mutant cDNA from pEXT-HRAS into pZIPneoSV(X) vector ²¹.
The pEF-FLAG-Memb-Dab clones were made by cloning SfiI-NotI fragments of pEF-Dab-VP16 into pEF-FLAG-Memb vector ⁹. All above constructs were verified by sequencing.

### Construction of Dab yeast libraries

The construction of the yeast pVP16*-Dab libraries was carried out as detailed elsewhere ¹³. The procedure comprises footprint mutagenesis to randomise CDR 2 and 3 of the VH segment of scFv625 (which comprised the canonical intrabody VH consensus framework ⁴ plus CDR1-CDR2-CDR3 of anti-RAS scFv33) or scFvI21R33 (which comprised a consensus framework from anti-RAS scFvI21R33) ⁹. These templates were sub-cloned into pVP16* vector ^{22,23}. To achieve diversification of the libraries, the two VH domains were separately amplified by PCR using two pairs of oligonucleotides:
for template scFv625 (consensus VH), EFFP2 plus conCDR2R and conCDR2F plus rdmCDR3R
for template scFvI21R33 (I21 VH), EFFP2 plus 33CDR2R and 33CDR2F plus rdmCDR3R.
Primer sequences:-
Template scFv625
   EFFP2: 5'- GGAGGGGTTTTATGCGATGG-3', which anneals with EF-1α promoter region of pEF-VP16.
   conCDR2R: 5'-CAGAGTCTGCATAGTATGTMNNMNNMNNMNNMNNACTAATGACTGAAA CCCAC-3'.
   conCDR2F: 5'- ACATACTATGCAGACTCTGTG -3' which hybridises with a part of the primer conCDR2R
   rdmCDR3R: 5'-TCCCTGGCCCCAGTAGTCAAA(MNNMNN)nCCCTCTCGCACAGTAATAG-3'(where n was varied to be 1 to 6 to give CDR3 variable length and to randomised CDR3.
Template scFvI21R33
   EFFP2: 5'- GGAGGGGTTTTATGCGATGG-3'.
   33CDR2R: 5'-CAGAGTCTGCATAGTATMNNMNNMNNMNNMNNACTAATGTATGAAA CCCAC-3'.
   33CDR2F: 5'- ATATACTATGCAGACTCTG -3'.
   rdmCDR3R: 5'-TCCCTGGCCCCAGTAGTCAAA(MNNMNN)nCCCTCTCGCACAGTAATAG-3'.

The amplification products were separated on agarose gels, purifie and a second PCR amplification carried out using EFFP2 plus JH5R (5'-GGTGACCAGGGTTCCCTGGCCCCAGTAGTC-3'), in which the two fragments were assembled and amplified. A final nested PCR was performed using EFFP and NotVHJR1 (which incorporates a NotI restriction site). The final PCR product was digested with SfiI plus Not1I and ligated into yeast pVP16* vector to yield the two pVP16*-Dab libraries 1. Ligated DNA were electroporated in the E.coli ElectroMAX DH10B (Invitrogen). The diversities of I21R33-derived library 1 was 2 x 10⁶ and of the consensus library 1 was 1.4 x 10⁶ (i.e. 3.4 X 10⁶ total diversity).
Dab libraries 2 were constructed by randomising CDR1 from each the first Dab libraries, with a similar footprint mutagenesis strategy ¹³. The VH domain of each Dab library 1 were separately amplified by PCR using two pairs of oligonucleotides sFvVP16F plus rdmCDR1R and CDR1F plus VP162R.
sFvVP16F: 5'- TGGGTCCGCCAGGCTCCAGG -3', which hybridise with ADH1 promoter region of pVP16*
rdmCDR1R: 5'-CCTGGAGCCTGGCGGACCCAMNNCATMNNMNNMNNACTGAAGCTGAAT CCAGAGG-3' that randomises four amino acid residues in CDR1
CDR1F: 5'-TGGGTCCGCCAGGCTCCAGG-3' which hybridises with a part of rdmCDR1R
VP162R: which hybridises with VP16 activator domain of pVP16*.

The two PCR fragments were assembled, amplified using sFvVP16F and VP162R, digested with SfiI plus NotI and ligated into yeast pVP16* vector. The respective diversities of library 2 was 3.04 x 10⁷ for I21R33-derived library and 2.215 x 10⁷ consensus-derived library (i.e 5.25 x 10⁷ total diversity).
12 clones were randomly picked from each library and sequenced to verify the insert and the correct integration of CDRs.

### Intracellular antibody capture (IAC) screening of Dab libraries

The screening of synthetic Dab libraries were performed according to the protocol of intrabody capture (IAC) technology as described ^{4,9} (see also a link within the Laboratory of Molecular Biology website http://www.mrc-lmb.cam.ac.uk) but excluding the phage panning step. 500 µg of pBTM116-antigen and 1mg of pooled pVP16*-Dab library 1 or pooled pVP16*-Dab library 2 were co-transfected into S. cerevisiae L40. Positive clones were selected by using auxotrophic markers, Trp, Leu and His. Positive colonies were selected for His prototropy and confirmed by β-galactosidase (β-gal) activity by filter assay. For the selected individual clones, false positive clones were eliminated by re-testing of His independent growth and β-gal activation, using relevant and non-relevant bait vectors. Ten double positive clones were which showed most rapid blue colour development in β-gal filter assays were sequenced.

### Mammalian luciferase reporter assay

The procedure is described in detail previously ^{4,9}. Briefly, the scFv or Dab were cloned into the pEF-VP16 expression vector and the antigen into pM1 vector ²⁰. COS7 cells were transiently co-transfected with 500ng of pG5-Luc, 50ng of pRL-CMV, 500ng of pEF-scFv-VP16 or pEF-Dab-VP16 and 500ng of pM1-antigen bait with 8µl of LipofectAMINE^{™} transfection reagent (Invitrogen), according to Manufacture's instruction. 48 hours after transfection, the cells were washed, lysed and assayed using Dual-Luciferase Reporter Assay System (Promega) in a luminometer. Transfection efficiency was normalised with the Renilla luciferase activity. The data represent two experiments, each performed in duplicate. To verify the expression of scFv-VP16 or Dab-VP16 fusion proteins, the transfected COS7 cells were analysed by SDS-PAGE, followed by Western blot using anti-VP16 (Santa-Cruz Biotechnology, 14-5) monoclonal antibody as primary antibody and HRP-conjugated rabbit anti-mouse IgG antibody (Amersham-Pharmacia Biotech (APB)) as secondary antibody. The blots were visualised by ECL detection kit (APB).

### Mammalian two hybrid assay in CD4 and GFP reporter CHO cells

Chinese hamster ovary (CHO) cells were grown in Minimal Essential Medium α (α-MEM, Invitrogen) with 10% foetal calf serum, penicillin and streptomycin. FACS analysis using CHO-CD4 line ¹⁴ was performed as described previously ²⁴. To establish the CHO-GFP line, pG5GFP-Hyg vector ¹⁷ was transfected in CHO parental lines with LipofectAMINE^{™} and the cells were selected for 7 days in α-MEM containing 0.3mg/ml hygromycin. CHO-GFP stable clone 39a was chosen for further assay. For FACS assay, 3 x 10⁵ CHO-CD4 or CHO-GFP cells were seeded in 6 well plates day before transfection. 0.5 µg of pM1-antigen and 1 µg of pEF-VP16-scFv or Dab were co-transfected into the cells. 48 hours after transfection, cells were washed, dissociated and resuspend in PBS. For CHO-CD4 assay, induction of cell surface CD4 expression was detected by using anti-human CD4 antibody (Pharmingen) and FITC-conjugated anti-mouse IgG as second layer (Pharmingen). The relative fluorescence of CHO-CD4 or CHO-GFP cells were measured with a FACSCalibur (Becton Dickinson) and the data were analysed by the CELLQuest software.

### Purification of Dab fragments and affinity measurement

Dabs were expressed for *in vitro* assays from the bacterial periplasm as previously described ⁹ Dab fragments were cloned into pHEN2 vector containing pelB leader sequence for periplasmic expression and His-tag and myc-tag. Dabs were expressed in 1 litre of medium for 4 hours at 30°C. The cells were harvested and extracted in 10 ml of cold TES buffer (Tris-HCl pH 7.5, EDTA, and sucrose). After dialysis, Dab fragments were purified using immobilised metal ion affinity chromatography, concentrated using Centricon concentrators (YM-10, Amicon) and the aliquots were stored at -70°C. Protein concentration was measured using Bio-Rad Protein assay Kit (Bio-Rad) according to Manufacture's instruction. Affinities of scFv and Dab were determined using surface plasmon resonance previously described ⁹ on a BIAcore 2000 instrument (Pharmacia Biosensor). The kinetic rate constants, *kₒₙ* and *k_{off}*, were evaluated using software supplied by the Manufacturer. Kd values were calculated from *k_{off}* and *kₒₙ* rate constants (Kd = *k_{off}* / *kₒₙ*). All measurements were performed in duplicate.

### Transformation assays in NIH3T3 cells

Low passage NIH3T3 cells clone D4 (a kind gift from Dr C. Marshall) were seeded at 2 x 10⁵ cells per well in 6-well plates the day before transfection. For transfection, 2µg of pEF-FLAG-Memb-scFv or pEF-FLAG-Memb-Dab vector, 100ng of pZIPneoSV(X)-HRASG12V vector were used plus 12µl of LipofectAMINE^{™}. Two days after transfection, the cells were transferred to 10 cm plates. After reaching confluence, they were kept for two weeks in Dulbecco's modified Eagle's medium containing 5% donor calf serum and penicillin and streptomycin. Foci formation due to loss of contact inhibition was scored by staining the plates with crystal violet.

**Table 1. Affinity measurements of anti-RAS scFv and Dab using BIAcore.**

| scFv / IDab | Kₒₙ(M⁻¹s⁻¹) | K_{off} (s⁻¹) | K_{d} (nM) |
|---|---|---|---|
| 33* | 1.76 ± 1.41 x 10⁵ | 1.13 ± 0.16 x 10⁻³ | 9.97 ± 8.82 |
| I21R-33VHI21VL | 4.78 ± 0.95 x 10⁴ | 8.65 ± 0.78 x 10⁻⁴ | 18.19 ± 1.85 |
| Dab 33 | 1.25 ± 0.12 x 10⁴ | 1.44 ± 0.68 x 10⁻² | 90.13 ± 9.70 |
| IDab anti-RAS #3 | 5.66 ± 0.18 x 10³ | 1.04 ± 0.01 x 10⁻³ | 182.98 ± 7.19 |
| IDab anti-RAS #10 | 2.32 ± 1.17 x 10⁴ | 2.54 ± 0.34 x 10⁻³ | 121.45 ± 46.6 |
| IDab anti-RAS #12 | 273 ± 1.12 x 10⁴ | 7.05 ± 2.28 x 10⁻⁴ | 26.65 ± 2.90 |

Proteins were expressed in bacteria but the final yields of purified Dab proteins were rather low (up to 0.5 mg per 1 litre of culture). Presumable, this is because of 'stickiness' and aggregation of Dabs at high concentration due to the exposed hydrophobic VL interface ²⁵. Biosensor measurements were made using the BIAcore 2000. The table summarises the value of association rate (*Kon*) and the dissociation rate (*Koff*) and calculated equilibrium dissociation constants (Kd) by BIA-evaluation 2.1 software. At high Dab concentrations, non-specific interaction between Dab and antigen were detected slightly.

### References

1. Biocca, S., Pierandrei-Amaldi, P. & Cattaneo, A. Intracellular expression of anti-p21ras single chain Fv fragments inhibits meiotic maturation of xenopus oocytes. Biochem Biophys Res Commun 197, 422-7 (1993).
2. Tavladoraki, P. et al. Transgenic plants expressing a functional single-chain Fv antibody are specifically protected from virus attack. Nature 366, 469-472 (1993).
3. Rondon, I. J. & Marasco, W. A. Intracellular antibodies (intrabodies) for gene therapy of infectious diseases. Annu Rev Microbiol 51, 257-83 (1997).
4. Tse, E. et al. Intracellular antibody capture technology: application to selection of single chain Fv recognising the BCR-ABL oncogenic protein. J. Mol. Biol. 317, 85-94 (2002).
5. Visintin, M. et al. The intracellular antibody capture technology (IACT): towards a consensus sequence for intracellular antibodies. J Mol. Biol. 317, 73-83 (2002).
6. Collins, F. S. et al. New goals for the U.S. Human Genome Project: 1998-2003. Science 282, 682-689 (1998).
7. Proba, K., Worn, A., Honegger, A. & Pluckthun, A. Antibody scFv fragments without disulfide bonds made by molecular evolution. J Mol Biol 275, 245-53. (1998).
8. Worn, A. & Pluckthun, A. Mutual stabilization of VL and VH in single-chain antibody fragments, investigated with mutants engineered for stability. Biochemistry 37, 13120-7. (1998).
9. Tanaka, T. & Rabbitts, T. H. Intrabodies based on intracellular capture frameworks that bind the RAS protein with high affinity and impair oncogenic transformation. *in prep* (2002).
10. Ward, E. S., Gussow, D., Griffiths, A. D., Jones, P. T. & Winter, G. Binding activities of a repertoire of single immunoglobulin variable domains secreted from Escherichia coli. Nature 341, 544-6. (1989).
11. Davies, J. & Riechmann, L. Single antibody domains as small recognition units: design and in vitro antigen selection of camelized, human VH domains with improved protein stability. Protein Eng 9, 531-7. (1996).
12. Reiter, Y., Schuck, P., Boyd, L. F. & Plaksin, D. An antibody single-domain phage display library of a native heavy chain variable region: isolation of functional single-domain VH molecules with a unique interface. J Mol Biol 290, 685-98. (1999).
13. Tanaka, T., Chung, G. T. Y., Forster, A., Lobato, M. N. & Rabbitts, T. H. De novo synthesis of antibody gene fragments and diversification by footprint mutagenesis. in prep (2002).
14. Fearon, E. R. et al. Karyoplasmic interaction selection strategy: A general strategy to detect protein-protein interaction in mammalian cells. Proc. Natl. Acad. Sci. USA 89, 7958-7962 (1992).
15. Martineau, P., Jones, P. & Winter, G. Expression of an antibody fragment at high levels in the bacterial cytoplasm. J Mol Biol 280, 117-127 (1998).
16. Portner-Taliana, A. et al. In vivo selection of single-chain antibodies using a yeast two-hybrid system. J Immunol Methods 238, 161-72. (2000).
17. Shioda, T., Andriole, S., Yahata, T. & Isselbacher, K. J. A green fluorescent protein-reporter mammalian two-hybrid system with extrachromosomal maintenance of a prey expression plasmid: application to interaction screening. Proc Natl Acad Sci USA 97, 5220-5224. (2000).
18. Bartel, P. L., Chien, C.-T., Sternglanz, R. & Fields, S. Cellular Interactions in Development: A Practical Approach (ed. Hartley, D. A.) (Oxford University Press, Oxford, 1993).
19. Hollenberg, S. M., Sternglanz, R., Cheng, P. F. & Weintraub, H. Identification of a new family of tissue -specific basic helix-loop-helix proteins with a two-hybrid system. Molecular and Cellular Biology 15, 3813-3822 (1995).
20. Sadowski, I., Bell, B., Broad, P. & Hollis, M. GAL4 fusion vectors for expression in yeast or mammalian cells. Gene 118,137-141 (1992).
21. Cepko, C. L., Roberts, B. E. & Mulligan, R. C. Construction and applications of a highly transmissible murine retrovirus shuttle vector. Cell 37,1053-62. (1984).
22. Vojtek, A. B., Hollenberg, S. M. & Cooper, J. A. Mammalian Ras interacts directly with the serine/threonine kinase Raf. Cell 74, 205-14 (1993).
23. Visintin, M., Tse, E., Axelson, H., Rabbitts, T. H. & Cattaneo, A. Selection of antibodies for intracellular function using a two-hybrid in vivo system. Proc. Natl. Acad. Sci. USA 96, 11723-11728 (1999).
24. Tse, E. & Rabbitts, T. H. Intracellular antibody-caspase mediated cell killing: a novel approach for application in canter therapy. Proc. Natl. Acad. Sci. USA 97, 12266-12271 (2000).
25. Riechmann, L. & Muyldermans, S. Single domain antibodies: comparison of camel VH and camelised human VH domains, J Immunol Methods 231, 25-38. (1999).
26. Kabat, E. A., Wu, T. T., Perry, H. M., Gottesman, K. S. & Foeller, C. Sequences of proteins of immunological interest (National Institutes of Health, Bethesda, 1991).
27. Lefranc, M.-P. & Lefranc, G. The Immumglobulin Factsbook (Academic Press, London, 2001).

## Claims

1. A method for determining the ability of a immunoglobulin V_{H} single domain to bind to a target in an intracellular environment, comprising the steps of:
a) providing a first molecule and a second molecule, wherein stable interaction of the first and second molecules leads to the generation of a signal;
b) providing a single intracellular V_{H} immunoglobulin domain which is associated with the first molecule, said single immunoglobulin V_{H} domain being free of complementary immunoglobulin domains;
c) providing an intracellular target which is associated with the second molecule, such that association of the immunoglobulin V_{H} domain and the target leads to stable interaction of the first and second molecules and generation of the signal;
d) assessing the intracellular interaction between the immunoglobulin domain and the target by monitoring the signal;
wherein the V_{H} domain exhibits at least 85% homology to the consensus sequence shown in Figure 5a and depicted as SEQ ID No 3.

2. A method according to claim 1, wherein the first and/or second molecules are polypeptides.

3. A method according to claim 2, wherein the first and second molecules associate to form an active reporter molecule.

4. A method according to claim 3, wherein the active reporter molecule is selected from the group consisting of a transcription factor, an enzyme and a bioluminescent molecule.

5. A method according to claim 6 wherein the active reporter molecule is an enzyme and the method is performed in the presence of a substrate for the enzyme.

6. A method according to any one of claims 4 to 5, wherein the first and second molecules are domains of the active reporter molecule.

7. A method according to claim 6, wherein the first molecule is the activation domain of VP16 and the second molecule is the DNA-binding domain of LexA.

8. A method according to any preceding claim, wherein the signal is selected from the group consisting of a change in an optical properly and the activation of a reporter gene.

9. A method according to claim 8, wherein the signal allows the sorting of cells.

10. A method according to any preceding claim, wherein the immunoglobulin single domain is provided by expressing an immunoglobulin-encoding nucleic acid within the cell.

11. A method according to claim 10, wherein the immunoglobulin-encoding nucleic acid is obtained from a library of immunoglobulin-encoding nucleic acids.

12. A method according to claim 11, wherein the library is a library encoding a repertoire of immunoglobulins.

13. A method according to claim 11, wherein the library is constructed from nucleic acids isolated from an organism which has been challenged with an antigen.

14. A method according to claim 1, comprising the further step of:
e) isolating those immunoglobulin single domains which give rise to a signal.

15. A method according to claim 14, comprising the further step of
f) subjecting the selected immunoglobulin single domains to a functional intracellular assay.

16. A method according to any preceding claim, wherein one or both of the immunoglobulin single domain and the target, together with the first or second molecules, are provided in the form of nucleic acid constructs which are transcribed to produce said immunoglobulin and/or target together with said first or second molecules.

17. A method for preparing an immunoglobulin single domain suitable for use in a procedure according to claim 1, comprising the steps of:
(a) expressing a repertoire of immunoglobulin domain genes in a selection system and isolating those genes which encode immunoglobulin domains specific for a desired target;
(b) bringing the isolated genes into operative association with nucleic acids encoding a first molecule, wherein stable interaction of the first molecule with a second molecule generates a signal, in order to product a fusion polypeptide comprising the immunoglobulin domain and the first molecule;
wherein each immunoglobulin domain gene encodes;
(a) a single intracellular V_{H} immunoglobulin domain which exhibits at least 85% homology to the consensus sequence shown in Figure 5a and depicted as SEQ ID NO.3.

18. A library of immunoglobulin single domains operatively associated with a first molecule, wherein stable interaction of the first molecule and a second molecule leads to the veneration of a signal; wherein each immunoglobulin single domain comprise
(a) a single intracellular V_{H} immunoglobulin domain which exhibits at least 85% homology to the consensus sequence shown in Figure 5a and depicted as SEQ ID NO.3.

19. A library according to claim 18, wherein the first and/or second molecules are polypeptides.

20. A library according to claim 18 or claim 19, wherein the first and second molecules associate to form an active reporter molecule.

21. A library according to claim 20, wherein the active reporter molecule is selected from the group consisting of a transcription factor, an enzyme and a bioluminescent molecule.

22. A library according toady one of claims 20 to 21, wherein the first and second molecules are domains of the active reporter molecule.

23. A library according to claim 22, wherein the first molecule is the activation domain of VP16 and the second molecule is the DNA-binding domain of LexA.

24. A method for preparing an intracellular single domain immunoglobulin which binds to a target in an intracellular environment, comprising the steps of:
a) providing a first molecule and a second molecule, wherein stable interaction of the first and second molecules leads to the generation of a signal;
b) providing an intracellular immunoglobulin which is associated with the first molecule;
c) providing an intracellular target which is associated with the second molecule, such that association of the immunoglobulin and the target leads to stable interaction of the first and second molecules and generation of the signal;
d) assessing the intracellular interaction between the immunoglobulin and the target by monitoring the signal; and
e) selecting one or more immunoglobulins which interact with the target and isolating one or more single domain immunoglobulins therefrom;
wherein each single domain immunoglobulin comprises
(a) a single intracellular V_{H} immunoglobulin domain which exhibits at least 85% homology to the consensus sequence shown in Figure 5a and depicted as SEQ ID NO.3.

25. A method according to claim 24, further comprising the step of mutating the framework regions of the single domain immunoglobulin to enhance intracellular binding and/or stability.

## Patentansprüche

1. Verfahren zur Bestimmung der Fähigkeit einer Immunoglobulin-V_{H}-Einzeldomäne, an ein Ziel in einer intrazellulären Umgebung zu binden, umfassend die folgenden Schritte:
a) Bereitstellen eines ersten Moleküls und eines zweiten Moleküls, wobei eine stabile Interaktion der ersten und zweiten Moleküle zu der Erzeugung eines Signals führt;
b) Bereitstellen einer einzelnen, intrazellulären V_{H}-Immunglobulin-Domäne, die mit dem ersten Molekül assoziiert ist, wobei die einzelne, intrazelluläre V_{H}-Immunglobulin-Domäne frei von komplementären Immunglobulin-Domänen ist;
c) Bereitstellen eines intrazellulären Ziels, das mit dem zweiten Molekül assoziiert ist, so dass die Assoziation der Immunglobulin-V_{H}-Domäne und des Ziels zu einer stabilen Interaktion der ersten und zweiten Moleküle und zur Erzeugung des Signals führt;
d) Bewerten der intrazellulären Interaktion zwischen der Immunglobulindomäne und dem Ziel durch Überwachung des Signals;
wobei die V_{H}-Domäne mindestens 85% Homologie mit der Konsensus-Sequenz aufweist, die in Figur 5a dargestellt und als SEQ ID Nr 3 angegeben ist.

2. Verfahren nach Anspruch 1, wobei das erste und/oder zweite Molekül Polypeptide sind beziehungsweise ein Polypeptid ist.

3. Verfahren nach Anspruch 2, wobei das erste und zweite Molekül zur Bildung eines aktiven Reportermoleküls assoziieren.

4. Verfahren nach Anspruch 3, wobei das aktive Reportermolekül ausgewählt ist aus der Gruppe bestehend aus einem Transkriptionsfaktor, einem Enzym und einem biolumineszenten Molekül.

5. Verfahren nach Anspruch 6, wobei das aktive Reportermolekül ein Enzym ist und das Verfahren in Gegenwart eines Substrates für das Enzym durchgeführt wird.

6. Verfahren nach einem der Ansprüche 4 bis 5, wobei das erste und zweite Molekül Domäne des aktiven Reportermoleküls sind.

7. Verfahren nach Anspruch 6, wobei das erste Molekül die Aktivierungsdomäne von VP16 ist und das zweite Molekül die DNA-Bindungsdomäne von LexA ist.

8. Verfahren nach einem vorangehenden Anspruch, wobei das Signal ausgewählt ist aus der Gruppe bestehend aus einer Änderung in einer optischen Eigenschaft und der Aktivierung eines Reportergens.

9. Verfahren nach Anspruch 8, wobei das Signal das Sortieren von Zellen ermöglicht.

10. Verfahren nach einem vorangehenden Anspruch, wobei die Immunglobulin-Einzeldomäne durch Exprimieren einer Immunglobulin kodierenden Nukleinsäure innerhalb der Zelle bereitgestellt ist.

11. Verfahren nach Anspruch 10, wobei die Immunglobulin kodierende Nukleinsäure von einer Bibliothek von Immunglobulin kodierenden Nukleinsäuren erhalten wird.

12. Verfahren nach Anspruch 11, wobei die Bibliothek eine Bibliothek ist, die für ein Repertoire von Immunglobulinen kodiert.

13. Verfahren nach Anspruch 11, wobei die Bibliothek aus Nukleinsäuren konstruiert ist, die von einem Organismus isoliert sind, der mit einem Antigen belastet wurde.

14. Verfahren nach Anspruch 1, umfassend den weiteren Schritt des:
e) Isolierens jener Immunglobulin-Einzeldomäne, die ein Signal entstehen lassen.

15. Verfahren nach Anspruch 14, umfassend den weiteren Schritt des:
f) Unterziehens der gewählten Immunglobulin-Einzeldomäne einem funktionellen, intrazellulären Assay.

16. Verfahren nach einem vorangehenden Anspruch, wobei eine oder beide von der Immunglobulin-Einzeldomäne und dem Ziel, gemeinsam mit dem ersten oder zweiten Molekül, in der Form von Nukleinsäurekonstrukten bereitgestellt sind, die transkribiert werden, um das Immunglobulin und/oder Ziel gemeinsam mit dem ersten oder zweiten Molekül zu erzeugen.

17. Verfahren zur Herstellung einer Immunglobulin-Einzeldomäne, die zur Verwendung in einer Prozedur nach Anspruch 1 geeignet ist, umfassend die folgenden Schritte:
(a) Exprimieren eines Repertoires von Immunglobulin-Domäne-Genen in einem Selektionssystem und Isolieren jener Gene, die für Immunglobulin-Domäne kodieren, die für ein gewünschtes Ziel spezifisch sind;
(b) Bringen der isolierten Gene in eine operative Assoziation mit Nukleinsäuren, die für ein erstes Molekül kodieren, wobei eine stabile Interaktion des ersten Moleküls mit einem zweiten Molekül ein Signal erzeugt, um ein Fusionspolypeptid zu erzeugen, das die Immunglobulin-Domäne und das erste Molekül umfasst;
wobei jedes Immunglobulin-Domäne-Gen für Folgendes kodiert:
(a) eine einzelne, intrazelluläre V_{H}-Immunglobulin-Domäne, die mindestens 85% Homologie mit der Konsensus-Sequenz aufweist, die in Figur 5a dargestellt und als SEQ ID Nr 3 angegeben ist.

18. Bibliothek von Immunglobulin-Einzeldomänen, die operativ mit einem ersten Molekül assoziiert sind, wobei eine stabile Interaktion des ersten Moleküls und eines zweiten Moleküls zu der Erzeugung eines Signals führt; wobei jede Immunglobulin-Einzeldomäne umfasst
(a) eine einzelne, intrazelluläre V_{H}-Immunglobulin-Domäne, die mindestens 85% Homologie mit der Konsensus-Sequenz aufweist, die in Figur 5a dargestellt und als SEQ ID Nr 3 angegeben ist.

19. Bibliothek nach Anspruch 18, wobei das erste und/oder zweite Molekül Polypeptide sind beziehungsweise ein Polypeptid ist.

20. Bibliothek nach Anspruch 18 oder Anspruch 19, wobei das erste und zweite Molekül zur Bildung eines aktiven Reportermoleküls assoziieren.

21. Bibliothek nach Anspruch 20 wobei das aktive Reportermolekül ausgewählt ist aus der Gruppe bestehend aus einem Transkriptionsfaktor, einem Enzym und einem biolumineszenten Molekül.

22. Bibliothek nach einem der Ansprüche 20 bis 21, wobei das erste und zweite Molekül Domäne des aktiven Reportermoleküls sind.

23. Bibliothek nach Anspruch 22, wobei das erste Molekül die Aktivierungsdomäne von VP16 ist und das zweite Molekül die DNA-Bindungsdomäne von LexA ist.

24. Verfahren zur Herstellung eines intrazellulären Einzeldomäne-Immunglobulins, das an ein Ziel in einer intrazellulären Umgebung bindet, umfassend die folgenden Schritte:
a) Bereitstellen eines ersten Moleküls und eines zweiten Moleküls, wobei eine stabile Interaktion der ersten und zweiten Moleküle zu der Erzeugung eines Signals führt;
b) Bereitstellen eines intrazellulären Immunglobulins, das mit dem ersten Molekül assoziiert ist;
c) Bereitstellen eines intrazellulären Ziels, das mit dem zweiten Molekül assoziiert ist, so dass die Assoziation des Immunglobulins und des Ziels zu einer stabilen Interaktion der ersten und zweiten Moleküle und zur Erzeugung des Signals führt;
d) Bewerten der intrazellulären Interaktion zwischen dem Immunglobulin und dem Ziel durch Überwachung des Signals; und
e) Selektieren eines oder mehrerer Immunglobuline, die mit dem Ziel interagieren, und Isolieren eines oder mehrerer Einzeldomäne-Immunglobuline daraus;
wobei jedes Einzeldomäne-Immunglobulin umfasst
(a) eine einzelne, intrazelluläre V_{H}-Immunglobulin-Domäne, die mindestens 85% Homologie mit der Konsensus-Sequenz aufweist, die in Figur 5a dargestellt und als SEQ ID Nr 3 angegeben ist.

25. Verfahren nach Anspruch 24, des Weiteren umfassend den Schritt des Mutierens der Framework-Regionen des Einzeldomäne-Immunglobulins zur Verstärkung der intrazellulären Bindung und/oder Stabilität.

## Revendications

1. Procédé de détermination de la capacité d'un domaine unique V_{H} d'immunoglobuline à se lier à une cible dans un environnement intracellulaire, le procédé comprenant les étapes consistant à :
a) fournir une première molécule et une deuxième molécule, l'interaction stable des première et deuxième molécules conduisant à la génération d'un signal ;
b) fournir un domaine unique V_{H} d'immunoglobuline intracellulaire qui est associé à la première molécule, ledit domaine unique V_{H} d'immunoglobuline étant dépourvu de domaines complémentaires d'immunoglobuline ;
c) fournir une cible intracellulaire qui est associée à la deuxième molécule, de sorte que l'association du domaine V_{H} d'immunoglobuline et de la cible conduise à l'interaction stable des première et deuxième molécules et à la génération du signal ;
d) évaluer l'interaction intracellulaire entre le domaine d'immunoglobuline et la cible par surveillance du signal ;
le domaine V_{H} présentant une homologie d'au moins 85 % avec la séquence consensus présentée sur la figure 5a et décrite comme SEQ ID NO : 3.

2. Procédé selon la revendication 1, dans lequel les première et/ou deuxième molécules sont des polypeptides.

3. Procédé selon la revendication 2, dans lequel les première et deuxième molécules s'associent pour former une molécule rapporteur active.

4. Procédé selon la revendication 3, dans lequel la molécule rapporteur active est choisie dans le groupe constitué par un facteur de transcription, une enzyme et une molécule bioluminescente.

5. Procédé selon la revendication 4, dans lequel la molécule rapporteur active est une enzyme et le procédé est réalisé en présence d'un substrat pour l'enzyme.

6. Procédé selon l'une quelconque des revendications 4 à 5, dans lequel les première et deuxième molécules sont des domaines de la molécule rapporteur active.

7. Procédé selon la revendication 6, dans lequel la première molécule est le domaine d'activation de VP16 et la deuxième molécule est le domaine de liaison à l'ADN de LexA.

8. Procédé selon l'une quelconque des revendications précédentes, dans lequel le signal est choisi dans le groupe constitué par une modification des propriétés optiques et l'activation d'un gène rapporteur.

9. Procédé selon la revendication 8, dans lequel le signal permet le tri des cellules.

10. Procédé selon l'une quelconque des revendications précédentes, dans lequel le domaine unique d'immunoglobuline est fourni par l'expression d'un acide nucléique codant pour une immunoglobuline à l'intérieur de la cellule.

11. Procédé selon la revendication 10, dans lequel l'acide nucléique codant pour l'immunoglobuline est obtenu d'une banque d'acides nucléiques codant pour une immunoglobuline.

12. Procédé selon la revendication 11, dans lequel la banque est une banque codant pour un répertoire d'immunoglobulines.

13. Procédé selon la revendication 11, dans lequel la banque est construite à partir d'acides nucléiques isolés d'un organisme qui a été exposé à un antigène.

14. Procédé selon la revendication 1, qui comprend l'étape supplémentaire consistant à :
e) isoler les domaines uniques d'immunoglobuline qui donnent lieu à un signal.

15. Procédé selon la revendication 14, qui comprend l'étape supplémentaire consistant à :
f) soumettre les domaines uniques d'immunoglobuline choisis à un test intracellulaire fonctionnel.

16. Procédé selon l'une quelconque des revendications précédentes, dans lequel l'un ou l'autre ou les deux du domaine unique d'immunoglobuline et de la cible, conjointement à la première molécule ou à la deuxième molécule, sont fournis sous la forme de constructions d'acides nucléiques qui sont transcrites pour produire ladite immunoglobuline et/ou ladite cible conjointement à ladite première molécule ou à ladite deuxième molécule.

17. Procédé de préparation d'un domaine unique d'immunoglobuline adapté pour être utilisé dans une procédure selon la revendication 1, le procédé comprenant les étapes consistant à :
(a) exprimer un répertoire de gènes de domaines d'immunoglobuline dans un système de sélection et isoler les gènes qui codent pour des domaines d'immunoglobulines spécifiques d'une cible souhaitée ;
(b) associer de manière fonctionnelle les gènes isolés aux acides nucléiques codant pour une première molécule, l'interaction stable de la première molécule avec une deuxième molécule générant un signal, de manière à produire un polypeptide de fusion comprenant le domaine d'immunoglobuline et la première molécule ;
chaque gène de domaine d'immunoglobuline codant pour :
(a) un domaine unique V_{H} d'immunoglobuline intracellulaire qui présente une homologie d'au moins 85 % avec la séquence consensus présentée sur la figure 5a et décrite comme SEQ ID NO : 3.

18. Banque de domaines uniques d'immunoglobuline associés de manière fonctionnelle à une première molécule, l'interaction stable de la première molécule et d'une et deuxième molécule conduisant à la génération d'un signal ; chaque domaine unique d'immunoglobuline comprenant :
(a) un domaine unique V_{H} d'immunoglobuline intracellulaire qui présente une homologie d'au moins 85 % avec la séquence consensus présentée sur la figure 5a et décrite comme SEQ ID NO : 3.

19. Banque selon la revendication 18, dans laquelle les première et/ou deuxième molécules sont des polypeptides.

20. Banque selon la revendication 18 ou la revendication 19, dans laquelle les première et deuxième molécules s'associent pour former une molécule rapporteur active.

21. Banque selon la revendication 20, dans laquelle la molécule rapporteur active est choisie dans le groupe constitué par un facteur de transcription, une enzyme et une molécule bioluminescente.

22. Banque selon l'une quelconque des revendications 20 à 21, dans laquelle les première et deuxième molécules sont des domaines de la molécule rapporteur active.

23. Banque selon la revendication 22, dans laquelle la première molécule est le domaine d'activation de VP16 et la deuxième molécule est le domaine de liaison à l'ADN de LexA.

24. Procédé de préparation d'une immunoglobuline intracellulaire à domaine unique qui se lie à une cible dans un environnement intracellulaire, le procédé comprenant les étapes consistant à :
a) fournir une première molécule et une deuxième molécule, l'interaction stable des première et deuxième molécules conduisant à la génération d'un signal ;
b) fournir une immunoglobuline intracellulaire qui est associée à la première molécule ;
c) fournir une cible intracellulaire qui est associée à la deuxième molécule, de sorte que l'association de l'immunoglobuline et de la cible conduise à l'interaction stable des première et deuxième molécules et à la génération du signal ;
d) évaluer l'interaction intracellulaire entre l'immunoglobuline et la cible par surveillance du signal ; et
e) choisir une ou plusieurs immunoglobulines qui interagissent avec la cible et isoler une ou plusieurs immunoglobulines à domaine unique parmi celles-ci ;
chaque immunoglobuline à domaine unique comprenant .
(a) un domaine unique V_{H} d'immunoglobuline intracellulaire qui présente une homologie d'au moins 85 % avec la séquence consensus présentée sur la figure 5a et décrite comme SEQ ID NO : 3.

25. Procédé selon la revendication 24, qui comprend en outre l'étape consistant à muter les régions cadres de l'immunoglobuline à domaine unique pour améliorer la liaison et/ou la stabilité intracellulaires.
